(19) Europäisches Patentamt
European Patent Office
Office européen des brevets

(11) EP 3 644 948 B1

(12) # EUROPEAN PATENT SPECIFICATION

(45) Date of publication and mention of the grant of the patent:
**13.09.2023 Bulletin 2023/37**

(21) Application number: **18731785.4**

(22) Date of filing: **11.06.2018**

(51) International Patent Classification (IPC):
***A61K 8/44*** (2006.01) ***A61K 8/46*** (2006.01)
***A61Q 5/02*** (2006.01) ***A61K 8/73*** (2006.01)
***A61Q 19/10*** (2006.01) ***A61K 8/02*** (2006.01)

(52) Cooperative Patent Classification (CPC):
**A61Q 19/10; A61K 8/0225; A61K 8/44; A61K 8/466; A61K 8/738; A61Q 5/02;**
A61K 2800/31; A61K 2800/596

(86) International application number:
**PCT/EP2018/065376**

(87) International publication number:
**WO 2019/001940 (03.01.2019 Gazette 2019/01)**

(54) **ANHYDROUS SOLID COMPOSITION COMPRISING AN ISETHIONIC ACID DERIVATIVE, A GLUTAMIC ACID DERIVATIVE, AN AMPHOTERIC SURFACTANT AND FILLERS**

WASSERFREIE FESTE ZUSAMMENSETZUNG MIT EINEM ISETHIONSÄUREDERIVAT, EINEM GLUTAMINSÄUREDERIVAT, EINEM AMPHOTEREN TENSID UND FÜLLSTOFFEN

COMPOSITION SOLIDE ANHYDRE COMPRENANT UN DÉRIVÉ D’ACIDE ISÉTHIONIQUE, UN DÉRIVÉ D’ACIDE GLUTAMIQUE, UN TENSIOACTIF AMPHOTÈRE ET DES CHARGES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2017 FR 1756220**

(43) Date of publication of application:
**06.05.2020 Bulletin 2020/19**

(73) Proprietor: **L’OREAL**
**75008 Paris (FR)**

(72) Inventors:
- **SEGALEN-GUIRAUD, Audrey**
**31170 Tournefeuille (FR)**
- **LAVABLE, Nadine**
**93400 Saint-Ouen (FR)**

(74) Representative: **L’Oreal**
**Service D.I.P.I.**
**9, rue Pierre Dreyfus**
**92110 Clichy (FR)**

(56) References cited:
**FR-A1- 3 030 269**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to a composition which is in anhydrous solid form, comprising suitably selected anionic surfactants, and at least one amphoteric surfactant, and at least 20% by weight, preferably at least 25% by weight, relative to the total weight of the composition, of fillers, and to the use thereof for cleansing and/or removing makeup from keratin materials such as the skin and the hair.

Many cosmetic washing products are known in the body and/or hair hygiene field. They are generally intended for cleansing keratin materials while at the same time providing them with good cosmetic properties.

Conventional products for cleansing keratin materials, such as shower gels and shampoos, are usually in the form of more or less thickened liquids. However, these products can prove to be difficult to meter out; the more liquid they are, the greater their tendency to run through the fingers, making it difficult to meter them out and creating waste, and/or the greater their tendency to leak out of their packaging, which can be very bothersome when they come into contact with clothing, for example during moving.

In order to modify the texture, and in particular to make it more compact, conventional means consist in using gelling agents or thickeners, but this is often done to the detriment of the cosmetic effects of the composition. Furthermore, these gelling agents or thickeners can have the drawback of inhibiting foam development.

In addition, it has been noted that thicker compositions often require a lot of rinsing water in order to remove the surplus product on the hair. In many countries where access to water is restricted, the rinsing time and therefore the amount required to properly rinse off the product are key indicators of the use qualities of a composition.

For these reasons, it may be advantageous to provide foaming products in the solid form which do not comprise water or which comprise very little water and which, in addition, exhibit the advantage of preventing microbiological problems and thus of not requiring the use of preservatives.

For example, the document FR 2 779 648 teaches solid compositions in the form of a deformable paste based on specific pulverulent fillers and anionic surfactants. These compositions can only be obtained using an extruder, which can be restrictive in processing terms.

In order to overcome some of these problems, other solid cosmetic formulations have been proposed, in particular shower products or shampoos in the form of granules or of solid powder.

Mention may particularly be made of US 4 330 438 which describes shampoo concentrates in powder form comprising anionic surfactants and nonionic derivatives of galactomannan, which form shampoos after dilution with water.

Mention may also be made of EP 0 796 318 which describes solid compositions obtained by drying and granulation of surface-active aqueous pastes in the presence of solid fillers, such as wood flours.

Mention may also be made of WO 2009/153311 which describes granulated shampoos comprising at least one surfactant deposited on solid particles. In said document, an aqueous phase comprising the surfactant is brought into contact with a solid phase constituted of fillers, in such a way that the surfactant agglomerates on the fillers. This step can be carried out in a mixer-granulator, in a mixer or in a fluidized bed granulator. Patent application FR 2 969 925 describes, for its part, anhydrous cosmetic compositions for cleansing keratin materials, comprising the combination of a nonionic surfactant with an anionic surfactant and at least 25% by weight of fillers and/or of fibers. These compositions may be in deformable solid form, and also in pulverulent form.

Finally, patent application FR 3 030 269 describes anhydrous solid cosmetic compositions, in particle form, comprising at least 30% by weight of anionic surfactants, at least 5% by weight of amphoteric surfactants and at least 10% by weight of fillers, for cleansing keratin materials such as the hair or the skin.

However, the solid compositions thus prepared may disaggregate or disintegrate with difficulty in the presence of water, and do not always make it possible to obtain rapid initiation of foaming and/or a sufficient abundance of foam. They can also be difficult to remove by rinsing, and can leave residues on the skin or the hair, which may then impact the cosmetic or esthetic performance levels of the product.

There is therefore the need to have compositions for washing keratin materials which do not run and which are more compact, modelable and economical. The compositions desired must disaggregate or disintegrate easily, must be easy to apply to keratin materials, and must allow rapid initiation of foaming, i.e. the rapid obtaining of an appropriate and sufficiently abundant foam, when the composition is applied, generally by rubbing, to said keratin materials which have optionally been pre-wetted.

Furthermore, for some years, the cosmetics market has been marked by a very high demand for formulations comprising ingredients of natural origin. Consumers desire formulations devoid of chemicals, to which they prefer ingredients of natural origin which are renowned for their better tolerance and affinity with the skin and which are more environmentally friendly.

A search is thus underway to obtain foaming cleansing products comprising compounds of natural origin which are satisfactorily harmless with regard to keratin materials, which exhibit good microbiological preservation, and which are easy and pleasant to use while nevertheless having the properties required for foaming products, namely good mixing with water, rapid transformation into foam and good rinsing.

The term "natural compound" means a compound that is obtained directly from the earth or the soil, or from plants or animals, via, where appropriate, one or more physical processes, for instance milling, refining, distillation, purification or filtration.

The term compound "of natural origin" means a natural compound that has undergone one or more additional chemical or industrial treatments, giving rise to modifications that do not affect the essential qualities of this compound and/or a compound predominantly comprising natural constituents that may or may not have undergone transformations as indicated above.

Mention may be made, as nonlimiting example of additional chemical or industrial treatment bringing about modifications which do not affect the essential qualities of a natural compound, of those allowed by the controlling bodies such as Ecocert (Reference system for biological and ecological cosmetic products, January 2003), or defined in recognized handbooks in the field, such as Cosmetics and Toiletries Magazine, 2005, Vol. 120, 9:10. The Applicant Company has discovered that an anhydrous composition in solid form, comprising a specific combination of anionic surfactants, at least one amphoteric surfactant and a high content of fillers, makes it possible to achieve these objectives.

More specifically, a subject of the invention is a composition, preferably a cosmetic composition, in anhydrous solid form, in the form of particles, the water content of the composition being less than or equal to 5% by weight, comprising at least one anionic surfactant chosen from acylisethionic acids and acylisethionates, at least one anionic surfactant chosen from acylglutamic acids and acylglutamates, at least one amphoteric surfactant, and at least 20% by weight of fillers, relative to the total weight of the composition.

According to one particular embodiment of the invention, the composition comprises, as amphoteric surfactant, at least one betaine derivative.

Compositions in accordance with the invention are advantageously in the form of granules, the particle size of which is homogeneous.

The compositions in accordance with the invention are easier to meter out, which enables a lower loss of the product. They disaggregate or disintegrate easily, and dissolution in water is rapid. They are easy to apply to keratin materials, and allow rapid initiation of foaming, i.e. the rapid obtaining of an appropriate and sufficiently abundant foam, when the composition is applied, generally by rubbing, to said keratin materials which have optionally been pre-wetted, and also a good foam quality, in particular a creamy, smooth and abundant foam. After application, good persistence of the fragrance is obtained and, when the composition is applied to the skin, the skin is nice and soft.

Another subject of the invention is a process for cleansing or for removing makeup from keratin materials such as the skin, including the scalp, keratin fibers such as the eyelashes or the hair, and/or the lips, characterized in that a cosmetic composition as defined above is applied to said keratin materials.

The composition according to the invention is intended for topical application and thus comprises a physiologically acceptable medium. The term "physiologically acceptable medium" here means a medium that is compatible with keratin materials.

In the context of the present invention, the term "keratin material" especially means the skin, the scalp, keratin fibers such as the eyelashes, the eyebrows, head hair, bodily hair, the nails, and mucous membranes such as the lips, and more particularly the skin (body, face, area around the eyes, eyelids).

In the text hereinbelow, the expression "at least one" is equivalent to "one or more" and, unless otherwise indicated, the limits of a range of values are included in that range.

## Solid anhydrous composition

The composition according to the invention is in solid form, that is to say that it does not flow under its own weight.

The compositions are in pulverulent form, namely in the form of particles such as granules.

According to one particular embodiment of the invention, the composition is in the form of granules.

Preferably, the particles according to the invention are small fractionated objects formed from solid particles aggregated together, of variable shapes and sizes. They may be regular or irregular in shape. They may in particular have a spherical shape, a square shape, a rectangular shape, or an elongated shape such as rods.

In particular, the composition according to the invention is in the form of small fractionated objects, which can have varied shapes, generally a regular shape and preferably a spherical shape, even better still a well-calibrated (uniform) spherical shape.

Spherical particles are quite particularly preferred.

The size of the particles can be, in the largest dimension thereof, between 1 and 5000 μm, preferably between 50 and 5000 μm, even more preferentially between 100 and 3500 μm, and better still between 200 and 3000 μm.

The size of the particles can be determined by manual sieving or via a mechanical calibrator, and also by laser particle size analysis, using for example the Mastersizer 3000 model sold by the company Malvern.

This solid presentation form has numerous advantages, such as a better preservation of the homogeneity of the mixture, a better flow and a reduction in the amount of dust given off. Thus, it allows easier handling of the compositions

and also facilitated storage and transportation.

The composition according to the invention is anhydrous, i.e. it does not comprise water (0%) or, if it does comprise water, the water content is less than or equal to 5% by weight, in particular less than or equal to 2% by weight, or even less than or equal to 1% by weight, even better still less than or equal to 0.5% by weight, relative to the total weight of the composition.

## Surfactants

The anionic surfactant(s) chosen from acylisethionic acids and acylisethionates, the anionic surfactant(s) chosen from acylglutamic acids and acylglutamates, and the amphoteric surfactant(s) present in the composition of the invention are generally foaming.

Foaming surfactants are detergents and differ from emulsifying surfactants by their HLB (hydrophilic-lipophilic balance) value, the HLB being the ratio between the hydrophilic part and the lipophilic part in the molecule. The term "HLB" is well known to those skilled in the art and is described, for example, in "The HLB system. A time-saving guide to Emulsifier Selection" (published by ICI Americas Inc.; 1984).

The term "HLB" is well known to those skilled in the art, and denotes the hydrophilic-lipophilic balance of a surfactant. The HLB or hydrophilic-lipophilic balance of the surfactant(s) used according to the invention may be determined via the Griffin method or the Davies method.

The higher the value, the greater the solubility in water, and, conversely, the lower the value, the greater the affinity of the surfactant for oil.

The Griffin HLB value is defined in the publication J. Soc. Cosm. Chem. 1954 (Volume 5), pages 249-256 or the HLB determined experimentally and as described in the publication from the authors F. Puisieux and M. Seiller, entitled Galenica 5: Les systèmes dispersés [Galenics 5: Dispersed systems] - Volume I - Agents de surface et émulsions [Surface agents and emulsions] - Chapter IV - Notions de HLB et de HLB critique [Notions of HLB and critical HLB], pages 153-194 - paragraph 1.1.2. Détermination de HLB par voie expérimentale [Experimental determination of HLB], pages 164-180.

It is preferably the calculated HLB value that should be taken into account.

The calculated HLB is defined as being the following coefficient:

calculated HLB = 20 $\times$ molar mass of the hydrophilic part / total molar mass.

The Davies HLB consists in summing up the hydrophilic and lipophilic contributions made by each of the structural groups of the surfactant:

$$HLB = \sum HLB_{\text{hydrophilic groups}} - \sum HLB_{\text{hydrophobic groups}} + 7$$

Thus, the HLB of the surfactant is equal to the sum of the HLB values of the hydrophilic groups minus the sum of the HLB values of the hydrophobic groups plus 7.

HLB tables exist for the various standard groups, which may be found especially in the following treatise: Surfactants in Cosmetics, second edition, surfactant science series volume 68, edited by Martin M.Rieger & Linda D.Rhein, p. 134, table 4.

Reference may be made to Kirk-Othmer's Encyclopedia of Chemical Technology, volume 22, pages 333-432, 3rd Edition, 1979, Wiley, for the definition of the emulsifying properties and functions of surfactants, in particular pages 347-377 of this reference for the anionic and nonionic surfactants.

According to a particular embodiment of the invention, the HLB values of the surfactant(s) are determined according to the Davies method which defines a scale ranging from 0 to several tens (in general up to 50).

For emulsifying surfactants, the HLB generally ranges from 3 to 8 for the preparation of water-in-oil (W/O) emulsions and from 8 to 18 for the preparation of oil-in-water (O/W) emulsions, whereas foaming surfactants generally have an HLB of greater than 18 and better still greater than 20.

### Isethionic acid derivatives

The composition according to the invention comprises at least one anionic surfactant chosen from acylisethionic acids and acylisethionates and mixtures thereof, in particular cosmetically acceptable salts thereof. This or these anionic surfactant(s) can for example be chosen from acyl isethionic acid salts of which the hydrocarbon-based chain R1 of the acyl group R1 C=O is linear or branched and saturated or unsaturated, and comprises from 8 to 30 carbon atoms, preferably from 10 to 22 carbon atoms, and better still from 12 to 18 carbon atoms.

The acyl group can in particular be chosen from lauroyl, myristoyl, palmitoyl, stearoyl, olivoyl, cocoyl and oleoyl groups, and mixtures thereof.

The term "cosmetically acceptable salts" means that the hydrogen atom of the acid function of the isethionic acid is replaced with a cation $M^+$ , for example, chosen from ions of alkali metals such as Na, Li or K, preferably Na or K, from ions of alkaline-earth metals such as Mg and from ammonium groups, and mixtures thereof.

Mention may in particular be made of the compounds of the following formula:

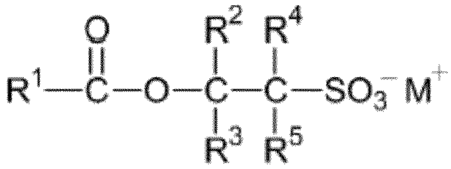

in which:

R1 represents a linear or branched, saturated or unsaturated hydrocarbon-based chain, comprising from 8 to 30 carbon atoms, preferably from 10 to 22 carbon atoms and better still from 12 to 18 carbon atoms,

$M^+$ represents $H^+$ or a cation, preferably chosen from ions of alkali metals such as Na, Li or K, from ions of alkaline-earth metals and from ammonium groups, and mixtures thereof, and

R2, R3, R4 and R5 represent, independently of one another, a hydrogen atom or a linear or branched alkyl group comprising from 1 to 4 carbon atoms.

Preferably, R1 represents a linear or branched alkyl group comprising from 10 to 20 carbon atoms, better still from 12 to 18 carbon atoms.

Preferably, R2 represents a hydrogen atom or a linear alkyl group comprising from 1 to 4 carbon atoms, such as methyl, ethyl, propyl or butyl, in particular a methyl group. Even more preferentially, R2 represents a hydrogen atom.

Preferably, R3 represents a hydrogen atom.

Preferably, R4 and R5 represent, independently of one other, a hydrogen atom or an alkyl group comprising from 1 to 4 carbon atoms. Even more preferentially, R4 and R5 represent a hydrogen atom.

As acylisethionates that may be used in the context of the invention, mention may for example be made of the compound bearing the INCI name sodium lauroyl methyl isethionate, for instance the references Iselux LQ-CLR, Iselux LQ-CLR-SB or the sodium lauroyl methyl isethionate/sodium methyl isethionate mixture such as Iselux (pellets) sold by the company Innospec. Mention may also be made of the compound bearing the INCI name sodium cocoyl isethionate, for instance the references Hostapon SCI sold by the company Clariant or Pureact I-85EC sold by the company Innospec Active Chemicals.

Mention may also be made of the sodium cocoyl isethionate sold under the name Jordapon CI P® by the company BASF.

The acylisethionic acids and acylisethionates may be present in the composition in a content of active material ranging from 1% to 60% by weight, preferably from 2% to 40% by weight, and better still from 5% to 30% by weight, relative to the total weight of the composition.

Glutamic acid derivatives

The composition in accordance with the invention comprises at least one anionic surfactant chosen from acylglutamic acids and acylglutamates, in particular cosmetically acceptable salts thereof.

According to one particular embodiment, they are chosen from acylglutamic acids in which the acyl group comprises from 10 to 30 carbon atoms, preferably from 12 to 22 carbon atoms, for instance lauroylglutamic acid, myristoylglutamic acid, palmitoylglutamic acid, stearoylglutamic acid, behenoylglutamic acid, olivoylglutamic acid, cocoylglutamic acid, and the salts of these acids, especially the salts of alkali metals such as Na, Li or K, preferably Na or K, the salts of alkaline-earth metals such as Mg, or the ammonium salts of said acids.

Mention may be made for example of the compounds bearing the INCI names lauroyl glutamic acid, cocoyl glutamic acid, sodium stearoyl glutamate, potassium lauroyl glutamate, potassium cocoyl glutamate and sodium olivoyl glutamate, and mixtures thereof.

Such compounds are sold under the name Amisoft by the company Ajinomoto and in particular under the references Amisoft CA, Amisoft LA, Amisoft HS 11 PF, Amisoft MK-11, Amisoft LK-11 and Amisoft CK-11, or alternatively under the name Eumulgin SG by the company Cognis.

Mention may also be made of the triethanolamine cocoyl glutamate sold under the name Amisoft CT 12 by the company Ajinomoto, the disodium cocoyl glutamate sold by the company Ajinomoto under the name Amisoft ECS-22SB, and the sodium lauroyl glutamate sold under the name Amisoft LS-11 by the company Ajinomoto. Mention may also be

made of the sodium cocoyl glutamate sold under the name Amisoft CS-11 by the company Ajinomoto.

As acylglutamic acid salt, mention may also be made of sodium hydrogenated tallowoyl glutamate, such as the product sold under the reference Acylglutamate GS 11 by the company Ajinomoto and disodium hydrogenated tallow glutamate, such as the product sold under the reference Acylglutamate HS-21 by the company Ajinomoto.

Mention may also be made of commercial mixtures of surfactants comprising at least one acylglutamic acids and acylglutamates, for instance the mixture of acyl glutamate salts such as Amisoft CS-22 sold by Ajinomoto.

According to one particular embodiment of the invention, the monosodium salt of N-lauroyl-L-glutamic acid, such as the product sold by the company Ajinomoto under the reference Amisoft HS 11 PF, is used.

The acylglutamic acids and acylglutamates may be present in the composition in accordance with the invention in a content of active material (AM) ranging from 1% to 60% by weight, preferably from 2% to 40% by weight, and better still from 5% to 30% by weight, relative to the total weight of the composition.

Amphoteric surfactants

The composition according to the invention also comprises one or more amphoteric surfactants.

The amphoteric surfactant(s) are chosen from amphoteric surfactants well known to those skilled in the art (this term including amphoteric and zwitterionic surfactants).

According to one particular embodiment of the invention, the amphoteric surfactant(s) are chosen from secondary or tertiary aliphatic, optionally quaternized, amine compounds comprising a linear or branched hydrocarbon-based chain containing from 8 to 22 carbon atoms, or a mixture of such hydrocarbon-based chains, and comprising at least one anionic group, such as for example a carboxylate, sulfonate or sulfate group.

The amphoteric surfactant(s) can be chosen from N-($C_8$-$C_{22}$)alkylbetaine, N-($C_8$-$C_{22}$)alkylsulfobetaine, N-($C_8$-$C_{22}$)alkylamido($C_1$-$C_6$)alkylbetaine, N-($C_8$-$C_{22}$)alkylsultaines, N-($C_8$-$C_{22}$)alkylhydroxysultaines, N-($C_8$-$C_{22}$)alkylamido($C_1$-$C_6$)alkylhydroxysultaines, N-($C_8$-$C_{22}$)alkylamphoacetates, N-($C_8$-$C_{22}$)alkylamphodiacetates, N-($C_8$-$C_{22}$)alkylamphopropionates, N-($C_8$-$C_{22}$)alkylamphodipropionates, N-($C_8$-$C_{22}$)alkylamphohydroxypropylsulfonates, diethylaminopropyl cocoaspartamide, and mixtures thereof.

These surfactants are in particular referenced in the catalog of the organization The Personnal Care Product Council.

The $C_8$-$C_{22}$ alkyl group can be chosen from octyl (or caprylyl), decyl (or capryl), lauryl, myristyl, palmityl, stearyl, arachidyl, behenyl groups, and mixtures thereof.

As N-($C_8$-$C_{22}$)alkylbetaine, mention may be particularly made of cocobetaine, such as the product sold under the name Dehyton AB-30® by the company BASF, decylbetaine (or decyl dimethyl glycine) , laurylbetaine (or lauryl dimethyl glycine), such as the product sold under the name Genagen KB® by the company Clariant, myristylbetaine (or myristyl dimethyl glycine), cetylbetaine (or cetyl dimethyl glycine), or stearylbetaine (or stearyl dimethyl glycine).

In a known manner, the coco group denotes the alkyl group deriving from coconut oil. It comprises predominantly a mixture of lauryl and myristyl groups.

Advantageously, the N-($C_8$-$C_{22}$)alkylbetaine are cocobetaine.

Among the N-($C_8$-$C_{22}$)alkylamido($C_1$-$C_6$)alkylbetaine, mention may be made for example of cocamidopropyl betaine (or cocamidopropyl dimethyl glycine), for example sold under the name Dehyton® PK 45 by the company BASF, or under the name Tego Betain CK D by the company Evonik Goldschmidt, the lauramidopropyl betaine sold under the name Rewoteric AMB12P® by the company Evonik Goldschmidt, myristamidopropyl betaine, stearamidopropyl betaine, capryl/capramidopropyl betaine, and palmitamidopropyl betaine.

Advantageously, the N-($C_8$-$C_{22}$)alkylamido($C_1$-$C_6$)alkylbetaine are chosen from N-($C_8$-$C_{22}$)alkylamidopropyl betaine, such as those mentioned previously, and are preferentially cocamidopropyl betaine.

As N-($C_8$-$C_{22}$)alkylsulfobetaine, mention may be made of lauryl sulfobetaine.

As N-($C_8$-$C_{22}$)alkylsultaines, mention may be made of capryl sultaine, lauryl sultaine, myristyl sultaine, and cocosultaine.

As N-($C_8$-$C_{22}$)alkylhydroxysultaines, mention may be made of capryl hydroxysultaine, lauryl hydroxysultaine (for example Betadet® S-20 from Kao), myristyl hydroxysultaine, coco hydroxysultaine and cetyl hydroxysultaine.

As N-($C_8$-$C_{22}$)alkylamido($C_1$-$C_6$)alkylhydroxysultaines, mention may be made of cocamidopropyl hydroxysultaine (such as the product sold under the name Amonyl 675 SB by the company SEPPIC or Betadet® SHR from Kao, lauramidopropyl hydroxysultaine, and myristamidopropyl hydroxysultaine.

As N-($C_8$-$C_{22}$)alkylamphoacetates, mention may be made for example of:

sodium N'-cocoyl-N-hydroxyethyl-N-carboxymethylethylenediamine (CTFA name: sodium cocamphoacetate);
sodium N'-caproyl-N-hydroxyethyl-N-carboxymethylethylenediamine (CTFA name: sodium caproamphoacetate);
sodium N'-capryloyl-N-hydroxyethyl-N-carboxymethylethylenediamine (CTFA name: sodium capryloamphoacetate);

sodium N'-isostearoyl-N-hydroxyethyl-N-carboxymethylethylenediamine (CTFA name: sodium isostearoamphoacetate);
sodium N'-lauroyl-N-hydroxyethyl-N-carboxymethylethylenediamine (CTFA name: sodium lauroamphoacetate);
sodium N'-myristoyl-N-hydroxyethyl-N-carboxymethylethylenediamine (CTFA name: sodium myristoamphoacetate);
sodium N'-palmitoyl-N-hydroxyethyl-N-carboxymethylethylenediamine (CTFA name: sodium palmamphoacetate);
sodium N'-stearoyl-N-hydroxyethyl-N-carboxymethylethylenediamine (CTFA name: sodium stearoamphoacetate).

As N-($C_8$-$C_{22}$)alkylamphodiacetates, mention may be made for example of:

- disodium N'-cocoyl-N-carboxymethoxyethyl-N-carboxymethylethylenediamine (CTFA name: disodium cocamphodiacetate), such as the product sold under the name Miranol C2M CONCENTRE NP® by the company Rhodia Chimie;
- disodium N'-caproyl-N-carboxymethoxyethyl-N-carboxymethylethylenediamine (CTFA name: disodium caproamphodiacetate);
- disodium N'-capryloyl-N-carboxymethoxyethyl-N-carboxymethylethylenediamine (CTFA name: disodium capryloamphodiacetate);
- disodium N'-isostearoyl-N-carboxymethoxyethyl-N-carboxymethylethylenediamine (CTFA name: disodium isostearoamphodiacetate);
- disodium N'-lauroyl-N-carboxymethoxyethyl-N-carboxymethylethylenediamine (CTFA name: disodium lauroamphodiacetate);
- disodium N'-stearoyl-N-carboxymethoxyethyl-N-carboxymethylethylenediamine (CTFA name: disodium stearoamphodiacetate).

Advantageously, the N-($C_8$-$C_{22}$)alkylamphodiacetates are disodium cocamphodiacetate. As N-($C_8$-$C_{22}$)alkylamphopropionates, mention may be made for example of:

sodium N'-cocoyl-N-hydroxyethyl-N-carboxyethylethylenediamine (CTFA name: sodium cocamphopropionate), such as the product sold under the name Rewoteric AM KSF 40 by the company Evonik Goldschmidt (code 52683);
sodium N'-caproyl-N-hydroxyethyl-N-carboxyethylethylenediamine (CTFA name: sodium caproamphopropionate);
sodium N'-capryloyl-N-hydroxyethyl-N-carboxyethylethylenediamine (CTFA name: sodium capryloamphopropionate);
sodium N'-isostearoyl-N-hydroxyethyl-N-carboxyethylethylenediamine (CTFA name: sodium isostearoamphopropionate);
sodium N'-lauroyl-N-hydroxyethyl-N-carboxyethylethylenediamine (CTFA name: sodium lauroamphopropionate);
sodium N'-stearoyl-N-hydroxyethyl-N-carboxyethylethylenediamine (CTFA name: sodium stearoamphopropionate).
As N-($C_8$-$C_{22}$)alkylamphodipropionates, mention may be made of: cocoamphodipropionic acid;
lauroamphodipropionic acid;
diethanolamine cocoamphodipropionate;
disodium N'-cocoyl-N-carboxyethoxyethyl-N-carboxyethylethylenediamine (CTFA name: disodium cocoamphodipropionate), such as the product sold under the name Mackam 2CSF 40 CG by the company Solvay;
disodium N'-caproyl-N-carboxyethoxyethyl-N-carboxyethylethylenediamine (CTFA name: disodium caproamphodipropionate);
disodium N'-capryloyl-N-carboxyethoxyethyl-N-carboxyethylethylenediamine (CTFA name: disodium capryloamphodipropionate);
disodium N'-isostearoyl-N-carboxyethoxyethyl-N-carboxyethylethylenediamine (CTFA name: disodium isostearoamphodipropionate);
disodium N'-lauroyl-N-carboxyethoxyethyl-N-carboxyethylethylenediamine (CTFA name: disodium lauroamphodipropionate).

As N-($C_8$-$C_{22}$ alkyl)amphohydroxypropylsulfonates, mention may be made of sodium caproamphohydroxypropylsulfonate, sodium capryloamphohydroxypropylsulfonate, sodium cocoamphohydroxypropylsulfonate, such as the product sold under the name Miranol CS CONCENTRE by the company Solvay, sodium lauroamphohydroxypropylsulfonate, and sodium stearoamphohydroxypropylsulfonate. The N-($C_8$-$C_{22}$)alkylamphoacetates, N-($C_8$-$C_{22}$)alkylamphodiacetates, N-($C_8$-$C_{22}$)alkylamphopropionates, N-($C_8$-$C_{22}$)alkylamphodipropionates and N-($C_8$-$C_{22}$ alkyl)amphohydroxypropylsulfonates described previously can be in free acid form or in salt form, in particular in the form of alkali metal (such as sodium or potassium) salts or alkaline-earth metal (calcium, magnesium) salts, and preferably in the form of sodium salt. Use may also be made of sodium monodiethylaminopropylcocoaspartamide, such as the product sold under

the name Chimexane HB by the company Chimex.

According to one particular embodiment of the invention, the amphoteric surfactant(s) may be chosen from N-alkyl($C_8$-$C_{22}$)betaines, N-alkyl($C_8$-$C_{22}$)amidoalkyl($C_6$-$C_8$)betaines, N-alkyl($C_8$-$C_{22}$)amphoacetates, N-alkyl($C_8$-$C_{20}$) amphodiacetates, and mixtures thereof. According to one preferred embodiment, the composition in accordance with the invention comprises at least one N-alkyl($C_8$-$C_{22}$)amidoalkyl($C_1$-$C_6$)betaine, preferably cocamidopropylbetaine.

The amphoteric surfactant(s) are generally present in the anhydrous composition according to the invention in an active material amount of between 0.5% and 40% by weight, relative to the total weight of the composition, in particular ranging from 1% to 30% by weight, better still from 1% to 20% by weight.

Additional surfactants

The composition in accordance with the invention can comprise additional surfactants different than the anionic surfactants and than the amphoteric surfactants such as those which are described above, but only insofar as the presence of these surfactants does not affect the comfort (innocuousness) of the composition. For the purposes of the present invention, these additional surfactants can also be foaming surfactants.

The additional surfactant(s), when they are present in the composition in accordance with the invention, can be chosen from anionic surfactants different than the acylisethionic acids and acylisethionates, than the acylglutamic acids and acylglutamates, and than salts thereof, nonionic surfactants and cationic surfactants. Preferably, they are chosen from anionic surfactants different than the isethionic acid derivatives, than the glutamic acid derivatives, and than salts thereof, and nonionic surfactants.

The nonionic surfactants can be chosen for example from phospholipids, alkyl polyglucosides (APGs), maltose esters, sucrose esters, hydrophobic gums, polyglycerolated fatty alcohols, fatty acid glycerol esters, oxyalkylenated glycerol esters, oxyalkylenated sugar esters, fatty acid polyethylene glycol esters, fatty acid sorbitan esters, glucamine derivatives such as 2-ethylhexyloxycarbonyl n-methylglucamine, and mixtures thereof.

The phospholipids used in the composition according to the invention may be of plant or animal origin and may be in pure form or in the form of a mixture.

The phospholipids used in the composition can in particular be lecithins, which are a complex mixture of phosphatides mainly chosen from phosphatidic acid, phosphatidylcholine, phosphatidylethanolamine, phosphatidylserine, lysophosphatidylcholine and/or phosphatidylinositol, in combination with variable amounts of other substances, such as triglycerides, glycolipids, sphingolipids, fatty acids and carbohydrates.

It will thus be possible to choose either the phospholipids mentioned above or the lecithins comprising these phospholipids.

According to a specific embodiment of the invention, the phospholipid or phospholipids are chosen from non-hydrogenated lecithins and hydrogenated lecithins.

Nonhydrogenated lecithins are generally obtained by lipid extraction, using nonpolar solvents, from plant or animal fatty substances. This lipid fraction usually predominantly comprises glycerophospholipids, including phosphatidylcholine.

The animal or plant sources that may be used to extract non-hydrogenated lecithins are, for example, soybean, sunflower or eggs. The glycerophospholipids included, in high proportion, in these lecithins are mainly phosphatidylcholine and phosphatidyl-ethanolamine.

The non-hydrogenated lecithins that are suitable for implementation of the present invention may be lecithins derived from soybean, from sunflower or from egg and/or mixtures thereof.

The lecithins are usually provided in a form dissolved in fatty acids, triglycerides or other solvents, or in the form of powders or cakes.

They are usually mixtures of lecithins, the content of glycerophospholipids of which, in the products as marketed, generally ranges from about at least 15% to about at least 95%.

Mention may be made, among the nonhydrogenated lecithins which may be suitable for the implementation of the cosmetic compositions in accordance with the present invention, of lecithins sold under the references Phospholipon 80®, Alcolec F100 And Phosale 75® by the company American Lecithin Company, Epikuron 145V by Cargill, Emulmetik 320 or 100J by Cargill, Ovothin 200 and Organic Lecithin which are sold by the company Lucas Meyer.

The hydrogenated lecithins are obtained by controlled hydrogenation of the nonhydrogenated lecithins as described above.

Mention may be made, as hydrogenated lecithins which can be used in the composition according to the invention, for example, of that which is sold under the reference Nikkol Lecinol S 10 by Nikko.

Use is preferably made, as alkyl polyglucosides, of those containing an alkyl group comprising from 6 to 30 carbon atoms and preferably from 8 to 16 carbon atoms and containing a hydrophilic (glucoside) group preferably comprising from 1.2 to 3 saccharide units. Examples that may be mentioned include decylglucoside (Alkyl-$C_9$/$C_{11}$-polyglucoside (1.4)), for instance the product sold under the name Mydol 10® by the company Kao, the product sold under

the name Plantaren 2000 UP® by the company BASF, and the product sold under the name Oramix NS 10® by the company SEPPIC; caprylyl/capryl glucoside, for instance the product sold under the name Oramix CG 110® by the company SEPPIC; laurylglucoside, for instance the products sold under the names Plantaren 1200 NO and Plantacare 1200® by the company BASF; cocoglucoside, for instance the product sold under the name Plantacare 818/UP® by the company BASF; cetostearyl glucoside optionally as a mixture with cetostearyl alcohol, sold, for example, under the name Montanov 68 by the company SEPPIC, under the name Tego-Care CG90 by the company Evonik Goldschmidt and under the name Emulgade KE3302 by the company BASF; arachidyl glucoside, for example in the form of the mixture of arachidyl alcohol and behenyl alcohol and arachidyl glucoside, sold under the name Montanov 202 by the company SEPPIC; cocoylethylglucoside, for example in the form of the mixture (35/65) with cetyl alcohol and stearyl alcohol, sold under the name Montanov 82 by the company SEPPIC; and mixtures thereof.

The oxyalkylenated glycerol esters are especially polyoxyethylenated derivatives of esters of glycerol and of a fatty acid and of their hydrogenated derivatives. These oxyalkylenated glycerol esters can be chosen, for example, from glyceryl esters of fatty acids which are hydrogenated and oxyethylenated, such as PEG-200 hydrogenated glyceryl palmate, sold under the name Rewoderm LI-S 80 by the company Evonik Goldschmidt; oxyethylenated glyceryl cocoates, such as PEG-7 glyceryl cocoate, sold under the name Tegosoft GC by the company Evonik Goldschmidt, and PEG-30 glyceryl cocoate, sold under the name Rewoderm LI-63 by the company Evonik Goldschmidt; and mixtures thereof.

The oxyalkylenated sugar esters are especially polyethylene glycol ethers of fatty acid and sugar esters. These oxyalkylenated sugar esters may be chosen, for example, from oxyethylenated glucose esters, such as PEG-120 methyl glucose dioleate, sold under the name Glucamate DOE 120 by the company Lubrizol.

The polyethylene glycol fatty acid esters are preferably $C_{16}$-$C_{22}$ fatty acid esters comprising from 8 to 100 ethylene oxide units.

The fatty chain of the esters can be chosen in particular from the stearyl, behenyl, arachidyl, palmityl or cetyl units and mixtures thereof, such as cetearyl, and preferably a stearyl chain. The number of ethylene oxide units may range from 8 to 100, preferably from 10 to 80, better still from 10 to 50. According to a particular embodiment of the invention, this number may range from 20 to 40.

As examples of fatty acid esters of polyethylene glycol, mention may be made of stearic acid esters respectively comprising 20, 30, 40, 50 and 100 ethylene oxide units, such as the products respectively sold under the names Myrj 49 P (polyethylene glycol 20 EO stearate; CTFA name: PEG-20 stearate), Myrj 51, Myrj 52 P (polyethylene glycol 40 EO stearate; CTFA name: PEG-40 stearate), Myrj 53 and Myrj 59 P by the company Croda. The esters of a $C_{16}$-$C_{22}$ fatty acid and of sorbitan are in particular esters of $C_{16}$-$C_{22}$ acids and of sorbitan and are formed by esterification with sorbitol of at least one fatty acid comprising at least one saturated or unsaturated linear alkyl chain respectively having from 16 to 22 carbon atoms. These esters may be chosen especially from sorbitan stearates, behenates, arachidates, palmitates or oleates, and mixtures thereof. Use is preferably made of sorbitan stearates and palmitates, and preferably sorbitan stearates.

As examples of sorbitan esters that may be used in the composition according to the invention, mention may be made of sorbitan monostearate (CTFA name: Sorbitan stearate), sold by the company Croda under the name Span 60, sorbitan tristearate, sold by the company Croda under the name Span 65 V, sorbitan monopalmitate (CTFA name: Sorbitan palmitate), sold by the company Croda under the name Span 40, sorbitan monooleate, sold by the company Croda under the name Span 80 V, and sorbitan trioleate, sold by the company Uniqema under the name Span 85 V. Preferably, the sorbitan ester used is sorbitan tristearate.

The esters of glycerol and of a fatty acid can be obtained in particular from an acid comprising a saturated linear alkyl chain having from 16 to 22 carbon atoms. Fatty acid esters of glycerol that may in particular be mentioned include glyceryl stearate (glyceryl mono-, di- and/or tristearate) (CTFA name: Glyceryl stearate) or glyceryl ricinoleate, and mixtures thereof. Preferably, the fatty acid ester of glycerol used is chosen from glyceryl stearates.

Mention may also be made of the mixture of glyceryl stearate and of polyethylene glycol 100 EO monostearate and in particular that comprising a 50/50 mixture sold under the name Arlacel 165 by the company Croda.

The fatty acid esters of sucrose are preferably chosen from esters derived from the reaction of sucrose(s) (saccharose) and of fatty acid(s) comprising from 10 to 24 carbon atoms, preferably from 12 to 20 carbon atoms, better still from 12 to 18 carbon atoms and even better still from 12 to 16 carbon atoms.

The fatty acids containing from 10 to 24 carbon atoms may be linear or branched, and saturated or unsaturated.

The fatty acids may be chosen from oleic acid, lauric acid, palmitic acid, myristic acid, stearic acid, linoleic acid and capric acid, or mixtures thereof.

According to one embodiment, the fatty acid ester of sucrose is chosen from esters derived from the reaction of sucrose and a fatty acid containing from 12 to 18 carbon atoms and preferably from 12 to 16 carbon atoms, such as lauric acid and/or palmitic acid, for instance sucrose laurate or sucrose palmitate, or a mixture thereof.

The fatty acid esters of sucrose may be chosen from mono-, di-, tri- and tetra-esters, and polyesters, and mixtures thereof. Esters with a low degree of esterification are preferably used, for instance fatty acid monoesters, diesters or triesters of sucrose, or a mixture thereof. The fatty acid ester of sucrose may be in the form of a mixture of

esters with a low degree of esterification, for instance a mixture of monoester and diester or a mixture of monoester, diester and triester.

In the case where use is made of a mixture of fatty acid esters of sucrose, preference is given to a mixture in which esters having a low degree of esterification, in particular the monoesters, are predominant and represent, for example, at least 50% by weight, preferably at least 60% by weight, of the mixture of fatty acid esters of sucrose.

Use may in particular be made of a mixture of esters of sucrose and of fatty acids comprising from 12 to 16 carbon atoms, in particular a mixture of mono-, di- and triesters of lauric acid or palmitic acid, it being possible for said mixture to comprise a minor amount (in a content of less than or equal to 40% by weight, relative to the weight of the mixture of fatty acid esters of sucrose) of fatty acid esters of sucrose in which the fatty acid comprises more than 16 carbon atoms.

Preferably, the fatty acid ester of sucrose which can be used in the present invention has an HLB value of greater than or equal to 10, preferably of greater than or equal to 12.

As is well known, the term HLB (Hydrophilic-Lipophilic Balance) means the equilibrium between the size and strength of the hydrophilic group and the size and strength of the lipophilic group of the surfactant.

The HLB value according to Griffin is defined in J. Soc. Cosm. Chem. 1954 (volume 5), pages 249-256.

By way of examples of fatty acid esters of sucrose or mixtures of fatty acid esters of sucrose, mention may be made of the products sold by the company Mitsubishi Kagaku Foods below:

- Surfhope SE Cosme C-1416, with an HLB value of 16, which is a sucrose myristate comprising about 80% monoester, the rest of the mixture being composed of di- and triesters,
- Surfhope SE Cosme C-1216, the INCI name of which is sucrose laurate, with an HLB value equal to 16, and which comprises from 75% to 90% monoester, the rest of the mixture being composed of di- and triesters,
- Surfhope SE Cosme C-1215L, the INCI name of which is sucrose laurate, with an HLB value equal to 15, comprising about 70% monoesters, the rest of the mixture being composed of diesters and other polyesters,
- Surfhope SE Cosme C-1616, with an HLB value of 16, which is a mixture of esters of sucrose and of palmitic and/or stearic acid (INCI name: sucrose palmitate), comprising from 75% to 90% monoester, the rest of the mixture being composed of di- and triesters, and possibly comprising sucrose stearate and sucrose palmitate stearate.

Mention may also be made of the ester of which the INCI name is sucrose laurate sold by the company Daiichi Kogyo Seiyaku under the reference DK ester S-L18A, with an HLB equal to 17, comprising 70% monoesters and 30% diesters and triesters.

As examples of esters or mixtures of esters of sucrose and of fatty acid, mention may also be made of:

- the products sold under the names F160, F140, F110, F90, F70 and SL40 by the company Crodesta, respectively denoting sucrose palmito-stearates formed of 73% of monoester and 27% of di- and triester, of 61% of monoester and 39% of di-, tri-, and tetraester, of 52% of monoester and 48% of di-, tri-, and tetraester, of 45% of monoester and 55% of di-, tri- and tetraester, of 39% of monoester and 61% of di-, tri- and tetraester, and sucrose monolaurate;
- the products sold under the name Ryoto Sugar Esters, for example referenced B370 and corresponding to the sucrose behenate formed of 20% of monoester and 80% of di-, tri- and polyester;
- the sucrose mono-dipalmito-stearate sold by the company Evonik Goldschmidt under the name Tegosoft PSE.

According to one embodiment, sucrose laurate is used.

According to one embodiment, the composition comprises at least one nonionic surfactant chosen from esters of sucrose, in particular sucrose laurate.

The amount of nonionic surfactants can range, for example, from 0.1 to 20% by weight, preferably from 0.5 to 15% by weight, better still from 1 to 10% by weight and even better still from 2 to 8% by weight, relative to the total weight of the composition.

The anionic surfactants different than the isethionic acid derivatives, than the glutamic acid derivatives, and than salts thereof can be chosen in particular from anionic derivatives of proteins of plant origin, amino acids and amino acid derivatives, alkyl sulfates, alkyl ether sulfates, sulfonates, taurates, sulfosuccinates, alkyl sulfoacetates, phosphates and alkyl phosphates, polypeptides, anionic derivatives of alkyl polyglucoside, soaps (salts of fatty acids), soybean oil derivatives, lactic acid derivatives, salts thereof and mixtures thereof. The anionic derivatives of proteins of plant origin are protein hydrolyzates bearing a hydrophobic group, it being possible for said hydrophobic group to be naturally present in the protein or to be added by reaction of the protein and/or of the protein hydrolyzate with a hydrophobic compound. The proteins are of plant origin and the hydrophobic group may especially be a fatty chain, for example an alkyl chain comprising from 10 to 22 carbon atoms.

As anionic derivatives of proteins of plant origin that may be used in the composition according to the invention, mention may be made more particularly of wheat, soybean, oat or silk protein hydrolyzates comprising an alkyl chain

containing from 10 to 22 carbon atoms, and salts thereof. The alkyl chain may especially be a lauryl chain and the salt may be a sodium, potassium and/or ammonium salt. Examples that may be mentioned include the sodium, potassium and/or ammonium salts of hydrolyzates of silk protein modified with lauric acid, such as the product sold under the name Kawa Silk by the company Kawaken; the sodium, potassium and/or ammonium salts of hydrolyzates of wheat protein modified with lauric acid, such as the potassium salt sold under the name Aminofoam W OR by the company Croda (CTFA name: potassium lauroyl wheat amino acids) and the sodium salt sold under the name Proteol LW 30 by the company SEPPIC (CTFA name: sodium lauroyl wheat amino acids); the sodium, potassium and/or ammonium salts of hydrolyzates of oat protein comprising an alkyl chain containing from 10 to 22 carbon atoms and more especially the sodium, potassium and/or ammonium salts of hydrolyzates of oat protein modified with lauric acid, such as the sodium salt sold under the name Proteol Oat (CTFA name: Sodium lauroyl oat amino acids); Proteol SAV 50S (INCI name: sodium cocoyl amino acid), Proteol APL (INCI name: sodium cocoyl apple amino acids) by the company SEPPIC, Amaranth S (INCI name: sodium cocoyl hydrolyzed amaranth proteins) by the company Lonza; and mixtures thereof.

Mention may be made, as alkyl ether sulfates, for example, of sodium lauryl ether sulfate (70/30 $C_{12}$-$C_{14}$) (2.2 EO), sold under the names Sipon AOS 225® or Texapon N702 Paste® by the company Cognis, ammonium lauryl ether sulfate (70/30 $C_{12}$-$C_{14}$) (3 EO), sold under the name Sipon LEA 370® by the company Cognis, or ammonium ($C_{12}$-$C_{14}$)alkyl ether (9 EO) sulfate, sold under the name Rhodapex AB/20® by the company Rhodia Chimie. Mention may be made, as sulfonates, for example, of $\alpha$-olefinsulfonates, such as the sodium $\alpha$-olefinsulfonate ($C_{14}$-$C_{16}$), sold under the name Bio-Terge AS-40® by the company Stepan, sold under the names Witconate AOS Protégé® and Sulframine AOS PH 12® by the company Witco or sold under the name Bio-Terge AS-40 CG® by the company Stepan, secondary sodium olefinsulfonate, sold under the name Hostapur SAS 30® by the company Clariant; or linear alkylarylsulfonates, such as sodium xylenesulfonate, sold under the names Manrosol SXS30®, Manrosol SXS40® and Manrosol SXS93® by the company Manro.

Mention may be made, as alkyl sulfoacetates, of lauryl sulfoacetate, such as, for example, that which is sold as a mixture with sodium methyl-2 sulfolaurate and disodium 2-sulfolaurate under the reference Stepan Mild PCL by the company Stepan.

Taurates that may be mentioned include the sodium salt of palm kernel oil methyltaurate sold under the name Hostapon CT Paste® by the company Clariant; N-acyl N-methyltaurates, for instance the sodium N-cocoyl N-methyltaurate sold under the name Hostapon LT-SF® by the company Clariant or sold under the name Nikkol CMT-30-T® by the company Nikko, or the sodium palmitoyl methyltaurate sold under the name Nikkol PMT® by the company Nikko.

Examples of sulfosuccinates that may be mentioned include oxyethylenated (3 EO) lauryl (70/30 $C_{12}$/$C_{14}$) alcohol monosulfosuccinate sold under the names Setacin 103 Special® by the company Z Schimmer & Schwarz and Rewopol SB-FA 30 K 4® by the company Evonik Goldschmidt, the disodium salt of a hemisulfosuccinate of $C_{12}$-$C_{14}$ alcohols, sold under the name Setacin F Special Paste® by the company Z Schimmer & Schwarz, the oxyethylenated (2 EO) disodium oleamidosulfosuccinate sold under the name Standapol SH 135® by the company Cognis, the oxyethylenated (5 EO) lauramide monosulfosuccinate sold under the name Lebon A-5000® by the company Sanyo, the disodium salt of oxyethylenated (10 EO) lauryl citrate monosulfosuccinate sold under the name Rewopol SB CS 50® by the company Evonik Goldschmidt, the disodium salt of lauryl alcohol monosulfosuccinate sold under the name Rewopol SB F12P® by the company Evonik Goldschmidt, or the ricinoleic monoethanolamide monosulfosuccinate sold under the name Rewoderm S 1333® by the company Witco.

As phosphates and alkyl phosphates, examples that may be mentioned include monoalkyl phosphates and dialkyl phosphates, such as lauryl monophosphate sold under the name MAP 20® by the company Kao, the potassium salt of dodecylphosphoric acid, a mixture of mono- and diester (predominantly diester) sold under the name Crafol AP-31® by the company Pulcra, the mixture of octylphosphoric acid monoester and diester, sold under the name Crafol AP-20® by the company Pulcra, the mixture of ethoxylated (7 mol of EO) 2-butyloctanol phosphoric acid monoester and diester, sold under the name Isofol 12 7 EO-Phosphate Ester® by the company Sasol, the potassium or triethanolamine salt of mono($C_{12}$-$C_{13}$)alkyl phosphate, sold under the references Arlatone MAP 230K-40® and Arlatone MAP 230T-60® by the company Uniqema, or the potassium lauryl phosphate sold under the name Dermalcare MAP XC-99/09® by the company Rhodia.

The anionic derivatives of alkyl polyglucosides can in particular be citrates, tartrates, sulfosuccinates, carbonates and glycerol ethers obtained from alkyl polyglucosides. Mention may be made, for example, of the sodium salt of cocoyl polyglucoside (1,4) tartaric ester, sold under the name Eucarol AGE-ET® by the company Lamberti, the disodium salt of cocoyl polyglucoside (1,4) sulfosuccinic ester, sold under the name Essai 512 MP® by the company SEPPIC, or the sodium salt of cocoyl polyglucoside (1,4) citric ester, sold under the name Eucarol AGE-EC® by the company Lamberti.

The soaps are obtained from a fatty acid which is partially or completely saponified (neutralized) with a basic agent. These are alkali metal or alkaline-earth metal soaps or soaps of organic bases. Use may be made, as fatty acids, of saturated, linear or branched fatty acids comprising from 8 to 30 carbon atoms and preferably comprising from 8 to 22 carbon atoms. This fatty acid may be chosen in particular from palmitic acid, stearic acid, myristic acid and lauric acid, and mixtures thereof.

Examples of basic agents that may be used include alkali metal hydroxides (sodium hydroxide and potassium hydroxide), alkaline-earth metal hydroxides (for example magnesium hydroxide), ammonium hydroxide or else organic bases, such as triethanolamine, N-methylglucamine, lysine and arginine. The soaps may especially be fatty acid alkali metal salts, the basic agent being an alkali metal hydroxide and preferably potassium hydroxide (KOH).

The amount of basic agent must be sufficient for the fatty acid to be at least partially neutralized.

Mention may especially be made of sodium or potassium laurate, potassium myristate, potassium palmitate, potassium stearate, potassium cocoate or KOH salts of stearic acid formed in situ.

The soybean oil derivatives and their salts are in particular the fatty acids and salts of fatty acids derived from soybean oil (the INCI name of which is "glycine soya oil" or "soybean oil") and in particular the salts of alkali metals, such as Na, Li or K, preferably Na or K, and of fatty acids resulting from soya, such as potassium soyate, such as, for example, that which is sold by the company Noveon.

As acylamino acids different than the glutamic acid derivatives, mention may for example be made of the sodium cocoyl glycinate sold by the company Ajinomoto under the name Amilite GCS-12, the analinates and derivatives thereof such as the product which is sold under the name Amilite ACS-12 by the company Amilon, the sodium cocoylglycinate sold by the company Ajinomoto under the name Amilite GCK-12, the sodium lauroyl sarcosinate sold by the company SEPPIC under the name Oramix L 30, the sodium and disodium stearoyl glutamates sold by the company Ajinomoto under the names Amisoft HS21 P and HS11 PF, and the sodium cocoyl sarcosinate sold by the company Zschimmer & Schwarz under the name Protelan LS 9011/C. Mention may also be made of the sodium salt of lauroyl oat amino acids, such as Proteol Oat sold by the company SEPPIC, or the compound of which the INCI name is sodium cocoyl amino acids, such as Proteol SAV 50 S from SEPPIC.

The amino acid derivatives may be chosen, for example, from sarcosinates and in particular acylsarcosinates, such as sodium lauroylsarcosinate sold under the name Oramix L 30® by the company SEPPIC, sodium myristoyl sarcosinate, sold under the name Nikkol Sarcosinate MN® by the company Nikko, or sodium palmitoyl sarcosinate, sold under the name Nikkol Sarcosinate PN® by the company Nikko; alaninates, such as sodium N-lauroyl-N-methylamidopropionate, sold under the name Sodium Nikkol Alaninate LN 30® by the company Nikko, and triethanolamine N-lauroyl-N-methylalanine, sold under the name Alanone Alta® by the company Kawaken Fine Chemicals; aspartates, such as the mixture of triethanolamine N-lauroyl aspartate and triethanolamine N-myristoyl aspartate, sold under the name Asparack® by the company Mitsubishi; and citrates.

The lactic acid derivatives or salts thereof may be chosen from acyl lactylic acid derivatives or salts thereof (lactylates), such as sodium stearoyl lactylate, provided for example by the company Aarhuskarl Shamn under the name Akoline SL, or else by Dr Straetmans under the name Dermofeel SL; sodium isostearoyl lactylate, such as the product sold by Rita under the name Pationic ISL; sodium behenoyl lactylate, for example sold by the company American Ingredients under the name Pationic SBL; sodium cocoyl lactylate, such as the product sold by the company Rita under the name Pationic SCL, sodium oleoyl lactylate, sodium lauroyl lactylate (Pationic 138C from Caravan Ingredients) or sodium caproyl lactylate (Capmul S8L-G from Abitec).

Mention may also be made of the sodium cocoamphoacetate, glycerol, lauryl glucoside, sodium cocoyl glutamate and sodium lauryl glucose carboxylate mixture sold by the company Cognis under the reference Plantapon SF.

The additional anionic surfactants can be present in a content ranging from 0.1 to 20% by weight, preferably from 0.5 to 15% by weight, better still from 1 to 10% by weight and even better still from 2 to 8% by weight, relative to the total weight of the composition.

According to a particular embodiment of the invention, the total amount of foaming surfactant active material is between 5% and 80% by weight, preferably between 10% and 60% by weight, and even more preferentially between 3% and 50% by weight of the total weight of the composition.

According to one particular embodiment of the invention, the composition comprises less than 5% by weight of anionic surfactant comprising at least one sulfate group, in particular less than 2% by weight, or even less than 1% by weight, even better still less than 0.5% by weight, relative to the total weight of the composition. Preferably, the composition is free of anionic surfactants comprising at least one sulfate group, for instance alkyl sulfates and alkyl ether sulfates.

## Fillers

The fillers are present in the composition according to the invention in a content of greater than or equal to 20% by weight, relative to the total weight of the composition, preferably greater than or equal to 25% by weight, better still greater than or equal to 45% by weight. The amount of fillers in the composition in accordance with the invention can range from 20% to 80% by weight, better still from 30% to 75% by weight, relative to the total weight of the composition.

The term "fillers" should be understood as meaning solid particles that are insoluble in the medium of the composition, irrespective of the temperature at which the composition is manufactured.

The fillers may be colorless or white and inorganic or organic, of any physical shape (platelet, spherical or

oblong) and of any crystallographic form (for example sheet, cubic, hexagonal, orthorhombic, etc.). The fillers may be porous or non-porous.

Mention may be made, as fillers, of inorganic fillers, such as silica, clays, ceramic beads, calcium carbonate, titanium oxides, talc or magnesium silicate (particle size: 5 microns), sold under the name Luzenac 15 M00® by the company Imerys, or talcs sold under the names Lyzenac 00 and Luzenac Pharma M by the company Imerys, kaolin or aluminum silicate, such as, for example, that sold under the name Kaolin Supreme® by Imerys, or sand with a particle size of between 1 and 1000 microns, or organic fillers, such as starches, in particular cornstarch, such as, for example, the product sold under the name Amidon de Mais B ® by the company Roquette, rice starch, Arrow root starch, and also derivatives thereof, Natpure Hollow Bead spheres sold by the company Sensient, celluloses such as the Cellulobead D-10 cellulose beads by the company Daitoka Seikogyo, colloidal silicas, sodium carbonate, Nylon microspheres, such as those sold under the name Orgasol 2002 UD NAT COS® by the company Atochem, micronized or nonmicronized plant powders, such as the fruit powders from Lessonia or bamboo powders, or rice grain husk powder, and mixtures thereof.

The starch derivatives used in the present invention may originate from a plant source such as cereals, tubers, roots, vegetables and fruit.

Thus, the starch(es) may originate from a plant source chosen from corn, pea, potato, sweet potato, banana, barley, wheat, rice, oat, sago, tapioca and sorghum.

Starches are generally in the form of a white powder, which is insoluble in cold water, of which the elemental particle size ranges from 15 to 100 microns.

In the context of the present invention, use may in particular be made of starches used in a form that has been crosslinked and in a form that has been chemically modified by functionalization with carboxyalkyl units.

The aim of the crosslinking is to form a network that is much more stable to heat and more resistant to heat and to acidity. The starch chains are linked to one another by bonding molecules: phosphated derivatives, chloroepoxide derivatives, acid dianhydrides and aldehyde derivatives.

The ($C_1$-$C_4$) carboxyalkyl starches, also referred to hereinafter as "carboxyalkyl starches", are obtained by grafting carboxyalkyl groups onto one or more alcohol functions of starch, in particular by reaction of starch and of sodium monochloroacetate in alkaline medium. The carboxyalkyl groups are generally attached via an ether function, more particularly to carbon 1.

The degree of substitution with carboxyalkyl units of the $C_1$-$C_4$ carboxyalkyl starch preferably ranges from 0.1 to 1 and more particularly from 0.15 to 0.5. The degree of substitution is defined according to the present invention as being the mean number of hydroxyl groups substituted with an ester or ether group per monosaccharide unit of the polysaccharide.

The carboxyalkyl starches are advantageously used in the form of salts and especially of salts of alkali metals or alkaline-earth metals such as Na, K, Li, $NH_4$, or salts of a quaternary ammonium or of an organic amine such as monoethanolamine, diethanolamine or triethanolamine.

The $C_1$-$C_4$ carboxyalkyl starches are advantageously, in the context of the present invention, carboxymethyl starches.

The carboxymethyl starches preferably comprise units having the following formula:

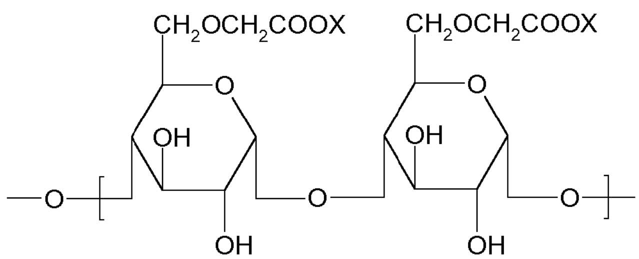

in which X, optionally covalently bonded to the carboxylic unit, denotes a hydrogen atom, an alkali metal or alkaline-earth metal such as Na, K, Li, $NH_4$, a quaternary ammonium or an organic amine, for instance monoethanolamine, diethanolamine or triethanolamine. Preferably, X denotes an Na+ cation.

The carboxyalkyl starches that may be used according to the present invention are partially or totally crosslinked carboxyalkyl starches.

In general, a crosslinked carboxyalkyl starch has, in contrast with a non-crosslinked carboxyalkyl starch, an increased, controllable viscosity of increased stability. The crosslinking thus makes it possible to reduce the syneresis phenomena and to increase the resistance of the gel to shear effects. It also makes it possible to increase the hydrophilicity of the material and also its disintegration rate.

The carboxyalkyl starches under consideration according to the invention are more particularly potato carboxyalkyl starches.

Thus, the carboxyalkyl starches that may be used according to the present invention are preferably sodium salts of carboxyalkyl starch, in particular a sodium salt of potato carboxymethyl starch, sold especially under the name Primojel by DMV International or Glycolys® and Glycolys LV® by the company Roquette.

According to a particular mode, use will be made of the potato carboxymethyl starches sold especially under the name Glycolys® by the company Roquette.

Fillers that may also be mentioned include exfoliant particles that allow scrubbing of the skin. Use may be made, as exfoliant particles, of exfoliant or scrubbing particles of mineral, plant or organic origins. Thus, it is possible to use, for example, polyethylene beads or powder, such as those sold under the name Microthene MN 727 or Microthene MN 710-20 by the company Equistar or such as the powder sold under the name Gotalene 120 Incolore 2 by the company Dupont; Nylon particles, such as those sold by the company Atochem under the name Orgasol 2002 Exd Nat Cos; polyvinyl chloride powder; pumice (INCI name: pumice), for instance ponce 3/B from Eyraud; ground shells of fruit kernels, such as ground apricot kernels or walnut shells; sawdust, wood flour, cork flour; glass beads; alumina (aluminum oxide) (INCI name: Alumina), such as the product sold under the name Dermagrain 900 by the company Marketech International; sugar crystals; beads which melt during application on the skin, such as, for example, spheres based on mannitol and cellulose which are sold under the Unisphere names by Induchem, agar-based capsules which are sold under the Primasponge names by the company Cognis and spheres based on jojoba esters which are sold under the Florasphères names by the company Floratech; and mixtures thereof.

According to one embodiment, the composition according to the invention comprises at least one filler chosen from talcs, starches, in particular rice starch and cornstarch, and mixtures thereof.

## Binding agents

According to one particular embodiment of the invention, the composition comprises at least one binding agent.

According to one preferred embodiment, the binding agent(s) are liquid, and hydrophilic or lipophilic, preferably hydrophilic (aqueous binders).

For the purposes of the present invention, the expression "hydrophilic or lipophilic liquid binding agent" is intended to mean a liquid which interacts with at least one solid starting material.

In the context of the invention, the binding agent(s) can be chosen from:

- polyols, such as glycerol, 1,3-propanediol, propylene glycol, butylene glycol, hexylene glycol, polyethylene glycols, such as PEG-8 or dipropylene glycol,
- sugars, such as mannitol, maltodextrin, sorbitol, xylitol, sucrose or glucose,

and mixtures thereof.

According to one particular embodiment of the invention, the composition comprises at least one binding agent chosen from polyols. It is preferably propylene glycol.

The binding agent(s) can represent from 1% to 30% by weight relative to the total weight of the composition, preferably from 2% to 20% by weight relative to the total weight of the composition, and at best 5% to 18%.

## Cyclodextrins

According to one particular embodiment of the invention, the composition comprises at least one cyclodextrin.

For the purposes of the present invention, the term "cyclodextrin" is intended to mean a cyclodextrin which is not chemically modified.

The cyclodextrins that can be used according to the present application are in particular oligosaccharides of formula:

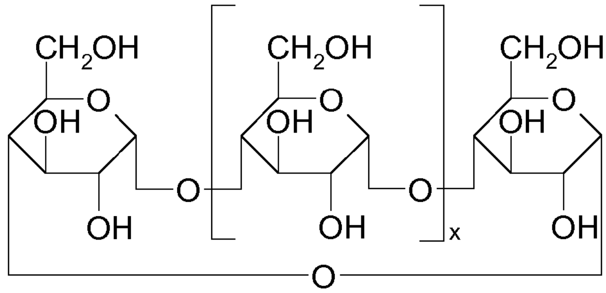

in which x may be a number equal to 4 (which corresponds to $\alpha$-cyclodextrin), to 5 ($\beta$-cyclodextrin) or to 6 ($\gamma$-cyclodextrin).

Preferably, the cyclodextrin in accordance with the invention is chosen from $\beta$-cyclodextrin and $\gamma$-cyclodextrin. It is preferably $\beta$-cyclodextrin.

Use may in particular be made of a $\beta$-cyclodextrin sold by the company Wacker under the name Cavamax W7 Pharma® and a $\gamma$-cyclodextrin sold by the company Wacker under the name Cavamax W8®.

The cyclodextrin(s) can represent from 1% to 70% by weight relative to the total weight of the composition, preferably from 2% to 30% by weight relative to the total weight of the composition, and at best 2% to 20%.

## Fatty acid salts

According to one particular embodiment of the invention, the composition comprises at least one fatty acid salt.

The fatty acid salt is advantageously a salt of a monocarboxylic acid comprising from 8 to 20 carbon atoms, preferably from 10 to 18 carbon atoms.

It is more particularly a salt of a fatty acid chosen from caprylic acid, capric acid, lauric acid, myristic acid, palmitic acid, stearic acid, oleic acid, or mixtures thereof such as cocoate salts.

The fatty acid salt can be chosen from alkali metal salts, alkaline-earth metal salts or amine salts. The salt can be chosen from sodium, potassium, calcium, magnesium, ammonium, diethanolamine, triethanolamine and triisopropanolamine salts.

As examples of fatty acid salts, mention may be made of:

- sodium or potassium caprate;
- sodium or potassium caprylate;
- sodium or potassium or magnesium or calcium or ammonium or triethanolamine laurate;
- sodium or potassium or magnesium or calcium or diethanolamine or triethanolamine or triisopropanolamine myristate;
- sodium or potassium or magnesium or triethanolamine palmitate;
- sodium or potassium or magnesium or triethanolamine cocoate;
- sodium or potassium or magnesium or ammonium or diethanolamine or triethanolamine stearate;
- sodium or potassium or ammonium oleate;
- sodium arachidate;
- sodium or potassium or calcium behenate.

Advantageously, use is made of a salt of a monocarboxylic acid having from 12 to 18 carbon atoms or mixtures thereof, such as those described previously. In particular, use may be made of a stearic acid salt.

According to one embodiment, the composition of the invention comprises magnesium stearate.

The fatty acid salt(s) may be present in the composition according to the invention in a content ranging from 0% to 5% by weight, preferably ranging from 0.1% to 2% by weight, relative to the total weight of the composition.

## Adjuvants

The compositions of the invention may contain adjuvants normally used in the cosmetics field and especially those used in cleansing products.

The compositions of the invention may in particular comprise at least one disintegrating agent. In the context of the present invention, the term "disintegrating agent" is intended to mean a constituent capable of accelerating the rate of separation of the particles forming the granules, for example in contact with water during the use of the composition. By way of examples, mention may for example be made of effervescent agents such as sodium bicarbonate and citric acid.

As adjuvants, mention may also be made of fragrances, preservatives, sequestering agents (EDTA, sodium

phytate), pigments, pearlescent agents, soluble dyes, sunscreens, cosmetic or dermatological active agents, such as water-soluble or fat-soluble vitamins, antiseptics, antiseborrheics, antimicrobials, such as benzoyl peroxide, salicylic acid, triclosan, azelaic acid, and also optical brighteners, nonionic polymers, such as polyvinylpyrrolidone (PVP), anionic polymers or fatty substances, such as oils or waxes. The amounts of these various adjuvants are those conventionally used in the field under consideration, for example from 0.01% to 20% of the total weight of the composition. These adjuvants and the concentrations thereof must be such that they do not modify the property desired for the composition of the invention.

As active agents, mention may be made of any care or cleansing active agent normally used in the cosmetics field, and in particular antibacterials, such as octopirox and triclosan, keratolytic agents, such as salicylic acid and derivatives thereof, lactic acid or glycolic acid, essential oils or vitamins, such as vitamin C (ascorbic acid), vitamin A (retinol), vitamin PP (niacinamide), vitamin B3 (panthenol) and derivatives thereof.

## Preparation process

According to one particular embodiment of the invention, the composition is in the form of granules.

Generally, the process used for obtaining these granules calls upon an agglomeration operation without compression, known as wet granulation. This granulation technique aims to convert materials which are generally pulverulent into small grains, by introducing a liquid which makes it possible to create bridges between the particles.

The agglomeration is carried out by introducing, into a shear granulator (for example: plowshare or vertical granulator) or rotary granulator (for example: granulating drum or plate mixer, or on a fluidized bed), on the one hand the pulverulent powder and, on the other hand, a small amount of a liquid which is generally water. This has the effect of bringing about the agglomeration of the particles with one another, thus resulting in the formation of agglomerates which take on a spherical shape by rolling and rubbing against one another.

Among the numerous factors which characterize the granules and the efficiency of the granulation operation, the main ones are the particle size and the particle distribution, and also the hardness of the granules obtained.

The use of one or more binding agents such as those which are defined above makes it possible to reduce the amount of water used and therefore the cost of the drying operation. According to one particular embodiment, all or part of the mixture is preprepared dry, that is to say that all of the powders are dry-mixed before granulation. The binder(s), alone or diluted in water or another solvent, or water, are then added to the pulverulent phase, then the granules obtained are dried, for example in a ventilated oven and/or under a fume hood or in a fluidized air bed. The particle size distribution is generally carried out by using sieves of different size or via a calibrator.

Wet granulation is constituted of the agglomeration of powders by means of a wetting liquid. The various steps of wet granulation are the following, it being possible for some of them to take place simultaneously:

- homogeneous mixing of the pulverulent starting materials in the granulator, for instance a Baker Perkins or Loedige granulator;
- preparation of the wetting solution, it being possible for this solution to contain at least one binding agent, and transfer into the granulator;
- wetting of the powders and mixing;
- granulation: the objective of this step is to mix powder and liquid so as to achieve the granular structure, by mechanical shear or by pulverization;
- drying, in order to reduce the moisture content of the granules. When the drying is carried out in an oven, a prior step consists in distributing the wet granule on a tray;
- calibration, which makes it possible to obtain a good particle size distribution by passing through a calibrated grill (sieve);
- optionally, addition of lubricants and/or disintegrating agents, the objective of the lubrication being to facilitate flow and to prevent sticking of the dry granule in the hoppers for feeding the packaging equipment and/or the forming machines, such as tablet presses. The compositions according to the invention can constitute in particular products for cleansing or removing makeup from the skin (body, face, eyes), scalp and/or hair.

Another subject of the invention is a process for cleansing or for removing makeup from keratin materials such as the skin, including the scalp, keratin fibers such as the eyelashes or the hair, and/or the lips, characterized in that a cosmetic composition as defined above is applied to said keratin materials.

According to one particular embodiment of the invention, the cosmetic process for cleansing keratin materials consists in applying the composition of the invention to keratin materials in the presence of water, and in removing the foam formed and the soiling residues by rinsing with water.

Also disclosed is the cosmetic use of the composition as defined above, as products for cleansing and/or removing makeup from keratin materials.

The examples that follow are given as illustrations of the invention and are not limiting in nature. All the amounts are given as weight percentages relative to the total weight of the composition. The names of the compounds are indicated where appropriate as chemical names or as INCI names.

## EXAMPLES

The compositions described in the examples are prepared by wet granulation according to the procedure below.

Procedure

The powders are mixed in a 1 l Baker Perkins granulator. The fragrance, then the binding agent, are added by means of a pipette, with stirring. The stirring is maintained so as to obtain granulation of the mixture by mechanical shear.
The granules thus obtained are distributed on a 160 micron sieve. They are subsequently placed in a ventilated oven at 50°C and then under a fume hood for drying.
The composition is then calibrated on a sieve in order to obtain a particle size of between 400 microns and 2500 microns.
The compositions thus obtained are then evaluated either qualitatively or quantitatively according to the tests below.

In vitro dispersion test

0.5 g of granules is mixed with 1 g of Hépar hard water at ambient temperature in a 15 ml graduated tube. The mixture is stirred for 10 seconds, in a Vortex-Genie 2 apparatus from Scientific Industries, at vortex speed 1.

In vitro foam test

0.5 g of granules is mixed with 1 g of Hépar hard water at ambient temperature in a 15 ml graduated tube. The mixture is stirred for 40 seconds, in a Vortex-Genie 2 apparatus from Scientific Industries, at vortex speed 1.
The amount of foam generated is measured in ml on the pipette.

**Example 1 according to the invention: shower granules**

The composition below is prepared.

| | | |
|---|---|---|
| Pulverulent phase | SODIUM COCOYL ISETHIONATE (PUREACT I-85 from INNOSPEC ACTIVE CHEMICALS or HOSTAPON SCI 85 from CLARIANT) | 16.65 |
| | SODIUM N-LAUROYL L-GLUTAMATE (AMISOFT LS 11 from AJINOMOTO) | 8.33 |
| | COCAMIDOPROPYLBETAINE POWDER (TEGO BETAIN CK D from EVONIK GOLDSCHMIDT) | 5.56 |
| | Beta-cyclodextrin (CAVAMAX W7 PHARMA from WACKER) | 10.17 |
| | Rice starch (REMY DR I (IRRADIATED) from BENEO-REMY) | 18.01 |
| | Talc (IMERCARE PHARMA from IMERYS) | 21.43 |
| | Dye(s) | qs |
| Liquid phase | Propylene glycol | 18.91 |
| | Fragrance(s) | 0.93 |

The composition above is evaluated qualitatively. It is in the form of granules, the particle size of which is homogeneous. It mixes well with water and readily disintegrates. It is easy to apply to the skin and the hair. It has good foaming properties: foam initiation is rapid, that is to say that an adequate and sufficiently abundant foam is rapidly obtained, and the foam quality is good; in particular, the foam is creamy and abundant.

**Comparative Example** 2

The composition below is prepared.

| Composition | | A | B | C |
|---|---|---|---|---|
| Pulverulent phase | SODIUM LAURYL SULFATE POWDER (TEXAPON Z 95 P from BASF) | 8.33 | 14.65 | 22.98 |
| | SODIUM COCOYL ISETHIONATE (PUREACT I-85 from INNOSPEC ACTIVE CHEMICALS or HOSTAPON SCI 85 from CLARIANT) | 16.65 | - | - |
| | SODIUM N-LAUROYL L-GLUTAMATE (AMISOFT LS 11 from AJINOMOTO) | - | 8.33 | - |
| | COCAMIDOPROPYLBETAINE POWDER (TEGO BETAIN CK D from EVONIK GOLDSCHMIDT) | 5.56 | 5.56 | 5.56 |
| | Beta-cyclodextrin (CAVAMAX W7 PHARMA from WACKER) | 10.17 | 12.17 | 12.17 |
| | Rice starch | 18.01 | 18.01 | 18.01 |
| | Talc (IMERCARE PHARMA from IMERYS) | 21.43 | 21.43 | 21.43 |
| | Dye(s) | qs | qs | qs |
| Liquid phase | Propylene glycol | 18.91 | 18.91 | 18.91 |
| | Fragrance(s) | 0.93 | 0.93 | 0.93 |

Dispersion results obtained

| Composition | Example 1 (invention) | A (comparative) | B (comparative) | C (comparative) |
|---|---|---|---|---|
| Dispersion | Instantaneous | Partial, slow | Partial, slow | Partial, slow |

Foam results obtained

| Composition | Example 1 (invention) | A (comparative) | B (comparative) | C (comparative) |
|---|---|---|---|---|
| Foam | 6 ml | 3 ml | 4 ml | 4 ml |

The composition of example 1 according to the invention disperses better and more rapidly and makes it possible to obtain more foam, under the hard water conditions at ambient temperature, than the comparative compositions A, B and C.

**Example 3 according to the invention: shower granules**

The composition below is prepared.

| | | |
|---|---|---|
| | SODIUM COCOYL ISETHIONATE | 17.43 |

(continued)

| | | |
|---|---|---|
| Pulverulent phase | (PUREACT I-85 from INNOSPEC ACTIVE CHEMICALS or HOSTAPON SCI 85 from CLARIANT) | |
| | SODIUM N-LAUROYL L-GLUTAMATE (AMISOFT LS 11 from AJINOMOTO) | 8.71 |
| | COCAMIDOPROPYLBETAINE POWDER (TEGO BETAIN CK D from EVONIK GOLDSCHMIDT) | 5.82 |
| | Beta-cyclodextrin (CAVAMAX W7 PHARMA from WACKER) | 10.65 |
| | Corn starch | 41.27 |
| | Dye(s) | qs |
| Liquid phase | Propylene glycol | 15.14 |
| | Fragrance(s) | 0.98 |

The composition above is evaluated qualitatively. It is in the form of granules, the particle size of which is homogeneous. It mixes well with water and readily disintegrates. It is easy to apply to the skin and the hair. It has good foaming properties: foam initiation is rapid, that is to say that an adequate and sufficiently abundant foam is rapidly obtained, and the foam quality is good; in particular, the foam is creamy and abundant.

**Example 4 according to the invention: shower granules**

The composition below is prepared.

| | | |
|---|---|---|
| Pulverulent phase | SODIUM COCOYL ISETHIONATE (PUREACT I-85 from INNOSPEC ACTIVE CHEMICALS or HOSTAPON SCI 85 from CLARIANT) | 16.65 |
| | SODIUM N-LAUROYL L-GLUTAMATE (AMISOFT LS 11 from AJINOMOTO) | 8.33 |
| | COCAMIDOPROPYLBETAINE POWDER (TEGO BETAIN CK D from EVONIK GOLDSCHMIDT) | 5.56 |
| | CROSSLINKED SODIUM CARBOXYMETHYL STARCH (POTATO) (GLYCOLYS from ROQUETTE) | 9.31 |
| | Rice starch | 18.01 |
| | Talc (IMERCARE PHARMA from IMERYS) | 22.30 |
| | Dye(s) | qs |
| Liquid phase | Propylene glycol | 18.91 |
| | Fragrance(s) | 0.93 |

The composition above is evaluated qualitatively. It is in the form of granules, the particle size of which is homogeneous. It mixes well with water and readily disintegrates. It is easy to apply to the skin and the hair. It has good foaming properties: foam initiation is rapid, that is to say that an adequate and sufficiently abundant foam is rapidly obtained, and the foam quality is good; in particular, the foam is creamy and abundant.

**Example 5 according to the invention: shower granules**

The composition below is prepared.

| | | |
|---|---|---|
| Pulverulent phase | SODIUM COCOYL ISETHIONATE (PUREACT I-85 from INNOSPEC ACTIVE CHEMICALS or HOSTAPON SCI 85 from CLARIANT) | 16.65 |
| | SODIUM N-LAUROYL L-GLUTAMATE (AMISOFT LS 11 from AJINOMOTO) | 8.33 |
| | COCAMIDOPROPYLBETAINE POWDER (TEGO BETAIN CK D from EVONIK GOLDSCHMIDT) | 5.56 |
| | Rice starch | 18.01 |
| | Talc (IMERCARE PHARMA from IMERYS) | 31.60 |
| | Dye(s) | qs |
| Liquid phase | Propylene glycol | 18.91 |
| | Fragrance(s) | 0.93 |

The composition above is evaluated qualitatively. It is in the form of granules, the particle size of which is homogeneous. It mixes well with water and readily disintegrates. It is easy to apply to the skin and the hair. It has good foaming properties: foam initiation is rapid, that is to say that an adequate and sufficiently abundant foam is rapidly obtained, and the foam quality is good; in particular, the foam is creamy and abundant.

## Claims

1. A composition in anhydrous solid form, in the form of particles, the water content of the composition being less than or equal to 5% by weight, comprising at least one anionic surfactant chosen from acylisethionic acids and acylisethionates, at least one anionic surfactant chosen from acylglutamic acids and acylglutamates, at least one amphoteric surfactant, and at least 20% by weight of fillers, relative to the total weight of the composition.

2. The composition as claimed in claim 1, wherein the at least one anionic surfactant is chosen from acylisethionic acids, acylisethionates and mixtures thereof, the hydrocarbon-based chain of the acyl group being linear or branched, and saturated or unsaturated, and comprising from 8 to 30 carbon atoms, preferably from 10 to 22 carbon atoms and better still from 12 to 18 carbon atoms.

3. The composition as claimed in either of claims 1 and 2, wherein the acylisethionates are chosen from the compounds of formula:

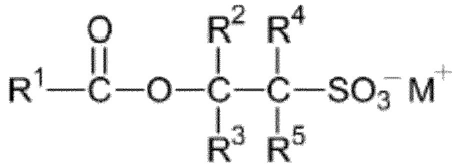

in which:

R1 represents a linear or branched, saturated or unsaturated hydrocarbon-based chain, comprising from 8 to 30 carbon atoms, preferably from 10 to 22 carbon atoms and better still from 12 to 18 carbon atoms,
$M^+$ represents $H^+$ or a cation, preferably chosen from ions of alkali metals such as Na, Li or K, from ions of alkaline-earth metals and from ammonium groups, and mixtures thereof, and
R2, R3, R4 and R5 independently represent a hydrogen atom or a linear or branched, preferably linear, alkyl group comprising from 1 to 4 carbon atoms.

4. The composition as claimed in one of claims 1 to 3, wherein the acylisethionates are chosen from sodium lauroyl methyl isethionate, sodium methyl isethionate, sodium cocoyl isethionate, and mixtures thereof.

5. The composition as claimed in any one of claims 1 to 4, wherein the acylisethionic acids and/or acylisethionates are present in an active material content ranging from 1% to 60% by weight, preferably from 2% to 40% by weight

and better still from 5% to 30% by weight, relative to the total weight of the composition.

6. The composition as claimed in any one of claims 1 to 5, wherein the acylglutamic acids and acylglutamates are chosen from acylglutamic acids in which the acyl group comprises from 10 to 30 carbon atoms, preferably from 12 to 22 carbon atoms, for instance lauroylglutamic acid, myristoylglutamic acid, palmitoylglutamic acid, stearoylglutamic acid, behenoylglutamic acid, olivoylglutamic acid, cocoylglutamic acid, and the salts of these acids, especially the salts of alkali metals such as Na, Li or K, preferably Na or K, the salts of alkaline-earth metals such as Mg, or the ammonium salts of said acids.

7. The composition as claimed in any one of claims 1 to 6, wherein the acylglutamic acids and acylglutamates are present in an active material content ranging from 1% to 60% by weight, preferably from 2% to 40% by weight and better still from 5% to 30% by weight, relative to the total weight of the composition.

8. The composition as claimed in one of claims 1 to 7, wherein the amphoteric surfactant(s) are chosen from N-($C_8$-$C_{22}$)alkylbetaines, N-($C_8$-$C_{22}$)alkylsulfobetaines, N-($C_8$-$C_{22}$)alkylamido($C_1$-$C_6$)alkylbetaines, N-($C_8$-$C_{22}$)alkylhydroxysultaines, N-($C_8$-$C_{22}$)alkylamido($C_1$-$C_6$)alkylhydroxysultaines, N-($C_8$-$C_{22}$)alkylamphoacetates, N-($C_8$-$C_{22}$)alkylamphodiacetates, N-($C_8$-$C_{22}$)alkylamphopropionates, N-($C_8$-$C_{22}$)alkylamphodipropionates, N-($C_8$-$C_{22}$)alkylamphohydroxypropylsulfonates, diethylaminopropyl cocoaspartamide, and mixtures thereof, preferably from N-alkyl($C_8$-$C_{20}$)betaines, N-($C_8$-$C_{20}$)alkylamido($C_1$-$C_6$)alkylbetaines, N-($C_8$-$C_{20}$)alkylamphoacetates and N-($C_8$-$C_{20}$)alkylamphodiacetates, and mixtures thereof.

9. The composition as claimed in any one of claims 1 to 8, comprising at least one N-($C_8$-$C_{22}$)alkylamido($C_1$-$C_6$)alkylbetaine.

10. The composition as claimed in one of claims 1 to 9, wherein the amphoteric surfactant(s) are present in an active material amount of between 0.5% and 40% by weight relative to the total weight of the composition, in particular ranging from 1% to 30% by weight, better still from 1% to 20% by weight.

11. The composition as claimed in any one of claims 1 to 10, wherein the fillers are present in a content of greater than or equal to 25% by weight, better still greater than or equal to 45% by weight, relative to the total weight of the composition.

12. The composition as claimed in any one of claims 1 to 11, wherein the amount of fillers ranges from 20% to 80% by weight, better still from 30% to 75% by weight, relative to the total weight of the composition.

13. The composition as claimed in any one of claims 1 to 12, comprising at least one filler chosen from talcs, starches, in particular rice starch and cornstarch, and mixtures thereof.

14. The composition as claimed in any one of claims 1 to 13, comprising at least one binding agent, preferably chosen from:

- polyols, such as glycerol, 1,3-propanediol, propylene glycol, butylene glycol, hexylene glycol, polyethylene glycols, such as PEG-8 or dipropylene glycol,
- sugars, such as mannitol, maltodextrin, sorbitol, xylitol, sucrose or glucose, and mixtures thereof.

15. The composition as claimed in any one of claims 1 to 14, comprising at least one binding agent chosen from polyols, preferably propylene glycol.

16. The composition as claimed in either one of claims 14 and 15, wherein the binding agent(s) represent from 1% to 30% by weight relative to the total weight of the composition, preferably from 2% to 20% by weight relative to the total weight of the composition, and at best 5% to 18%.

17. The composition as claimed in any one of claims 1 to 16, comprising at least one cyclodextrin.

18. The composition as claimed in claim 17, wherein the cyclodextrin(s) are oligosaccharides of formula:

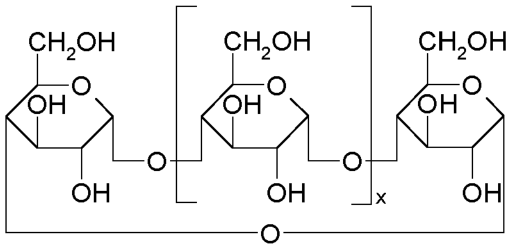

in which x may be a number equal to 4 (which corresponds to α-cyclodextrin), to 5 (β-cyclodextrin) or to 6 (γ-cyclodextrin).

**19.** The composition as claimed in either of claims 17 and 18, wherein the cyclodextrin(s) are chosen from β-cyclodextrin and γ-cyclodextrin; preferably β-cyclodextrin

**20.** The composition as claimed in any one of claims 17 to 19, wherein the cyclodextrin(s) represent from 1% to 70% by weight relative to the total weight of the composition, preferably from 2% to 30% by weight relative to the total weight of the composition, and at best 2% to 20%.

**21.** A process for cleansing or removing makeup from keratin materials, wherein a composition as defined in any one of claims 1 to 20 is applied to the keratin materials.

**22.** A process for preparing a composition as defined in any one of claims 1 to 20 by wet granulation, comprising the following steps, it being possible for some of them to take place simultaneously:

- homogeneous mixing of the pulverulent raw materials in the granulator;
- preparation of the wetting solution, it being possible for this solution to contain at least one binding agent, and transfer into the granulator;
- wetting of the powders and mixing;
- granulation: the objective of this step is to mix powder and liquid so as to achieve the granular structure, by mechanical shear or by pulverization;
- drying, in order to reduce the moisture content of the granules;
- calibration, which makes it possible to obtain a good particle size distribution by passing through a calibrated grill or sieve;
- optionally, addition of lubricants and/or disintegrating agents.

**Patentansprüche**

**1.** Zusammensetzung in wasserfreier fester Form, in Form von Partikeln, wobei der Wassergehalt der Zusammensetzung kleiner als oder gleich 5 Gew.-% ist, umfassend mindestens ein anionisches Tensid ausgewählt aus Acylisethionsäuren und Acylisethionaten, mindestens ein anionisches Tensid ausgewählt aus Acylglutaminsäure und Acylglutamaten, mindestens ein amphoteres Tensid und mindestens 20 Gew.-% Füllstoffe, bezogen auf das Gesamtgewicht der Zusammensetzung.

**2.** Zusammensetzung nach Anspruch 1, wobei das mindestens eine anionische Tensid aus Acylisethionsäuren, Acylisethionaten und Mischungen davon ausgewählt ist, wobei die Kohlenwasserstoffkette der Acylgruppe geradkettig oder verzweigt und gesättigt oder ungesättigt ist und 8 bis 30 Kohlenstoffatome, vorzugsweise 10 bis 22 Kohlenstoffatome und noch mehr bevorzugt 12 bis 18 Kohlenstoffatome umfasst.

**3.** Zusammensetzung nach Anspruch 1 oder 2, wobei die Acylisethionate ausgewählt sind aus den Verbindungen der Formel:

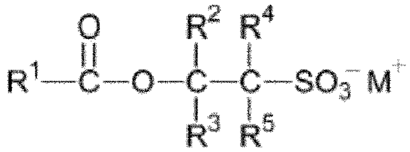

in denen:

R1 für eine geradkettige oder verzweigte, gesättigte oder ungesättigte Kohlenwasserstoffkette mit 8 bis 30 Kohlenstoffatomen, vorzugsweise 10 bis 22 Kohlenstoffatomen und besonders bevorzugt 12 bis 18 Kohlenstoffatomen steht,
$M^+$ für $H^+$ oder ein Kation, vorzugsweise ausgewählt aus Ionen von Alkalimetallen wie Na, Li oder K, aus Ionen von Erdalkalimetallen und aus Ammoniumgruppen, sowie Mischungen davon, steht und
R2, R3, R4 und R5 unabhängig voneinander für ein Wasserstoffatom oder eine geradkettige oder verzweigte, vorzugsweise geradkettige, Alkylgruppe mit 1 bis 4 Kohlenstoffatomen stehen.

4. Zusammensetzung nach einem der Ansprüche 1 bis 3, wobei die Acylisethionate aus Natriumlauroylmethylisethionat, Natriummethylisethionat, Natriumcocoylisethionat und Mischungen davon ausgewählt sind.

5. Zusammensetzung nach einem der Ansprüche 1 bis 4, wobei die Acylisethionsäuren und/oder Acylisethionate in einer Wirkstoffmenge im Bereich von 1 Gew.-% bis 60 Gew.-%, vorzugsweise 2 Gew.-% bis 40 Gew.-% und besonders bevorzugt 5 Gew.-% bis 30 Gew.-%, bezogen auf das Gesamtgewicht der Zusammensetzung, vorliegen.

6. Zusammensetzung nach einem der Ansprüche 1 bis 5, wobei die Acylglutaminsäuren und Acylglutamate aus Acylglutaminsäuren, in denen die Acylgruppe 10 bis 30 Kohlenstoffatome, vorzugsweise 12 bis 22 Kohlenstoffatome, umfasst, zum Beispiel Lauroylglutaminsäure, Myristoylglutaminsäure, Palmitoylglutaminsäure, Stearoylglutaminsäure, Behenoylglutaminsäure, Olivoylglutaminsäure, Cocoylglutaminsäure, und den Salzen dieser Säuren, insbesondere den Salzen von Alkalimetallen wie Na, Li oder K, vorzugsweise Na oder K, den Salzen von Erdalkalimetallen wie Mg oder den Ammoniumsalzen dieser Säuren, ausgewählt sind.

7. Zusammensetzung nach einem der Ansprüche 1 bis 6, wobei die Acylglutaminsäuren und Acylglutamate in einer Wirkstoffmenge im Bereich von 1 Gew.-% bis 60 Gew.-%, vorzugsweise 2 Gew.-% bis 40 Gew.-% und besonders bevorzugt 5 Gew.-% bis 30 Gew.-%, bezogen auf das Gesamtgewicht der Zusammensetzung, vorliegen.

8. Zusammensetzung nach einem der Ansprüche 1 bis 7, wobei das/die amphotere(n) Tensid(e) aus N-($C_8$-$C_{22}$)-Alkylbetainen, N-($C_8$-$C_{22}$)-Alkylsulfobetainen, N-($C_8$-$C_{22}$)-Alkylamido-($C_1$-$C_6$)-alkylbetainen, N-($C_8$-$C_{22}$)-Alkylhydroxysultainen, N-($C_8$-$C_{22}$)-Alkylamido-($C_1$-$C_6$)-alkylhydroxysultainen, N-($C_8$-$C_{22}$)-Alkylamphoacetaten, N-($C_8$-$C_{22}$)-Alkylamphodiacetaten, N-($C_8$-$C_{22}$)-Alkylamphopropionaten, N-($C_8$-$C_{22}$)-Alkylamphodipropionaten, N-($C_8$-$C_{22}$)-Alkylamphohydroxypropylsulfonaten, Diethylaminopropylcocoaspartamid und Mischungen davon, vorzugsweise aus N-Alkyl-($C_8$-$C_{20}$)-betainen, N-($C_8$-$C_{20}$)-Alkylamido-($C_1$-$C_6$)-alkylbetaines, N-($C_8$-$C_{20}$)-Alkylamphoacetaten und N-($C_8$-$C_{20}$)-Alkylamphodiacetaten und Mischungen davon ausgewählt ist/sind.

9. Zusammensetzung nach einem der Ansprüche 1 bis 8, umfassend mindestens ein N-($C_8$-$C_{22}$)-Alkylamido-($C_1$-$C_6$)-alkylbetain.

10. Zusammensetzung nach einem der Ansprüche 1 bis 9, wobei das/die amphotere(n) Tensid(e) in einer Wirkstoffmenge von 0,5 Gew.-% bis 40 Gew.-% bezogen auf das Gesamtgewicht der Zusammensetzung, insbesondere im Bereich von 1 Gew.-% bis 30 Gew.-%, besonders bevorzugt 1 Gew.-% bis 20 Gew.-%, vorliegen.

11. Zusammensetzung nach einem der Ansprüche 1 bis 10, wobei die Füllstoffe in einer Menge von mehr als oder gleich 25 Gew.-%, besonders bevorzugt mehr als oder gleich 45 Gew.-%, bezogen auf das Gesamtgewicht der Zusammensetzung, vorliegen.

12. Zusammensetzung nach einem der Ansprüche 1 bis 11, wobei die Füllstoffmenge im Bereich von 20 Gew.-% bis 80 Gew.-%, besonders bevorzugt 30 Gew.-% bis 75 Gew.-%, bezogen auf das Gesamtgewicht der Zusammensetzung, liegt.

13. Zusammensetzung nach einem der Ansprüche 1 bis 12, umfassend mindestens einen Füllstoff ausgewählt aus Talkum, Stärken, insbesondere Reisstärke und Maisstärke, und Mischungen davon.

14. Zusammensetzung nach einem der Ansprüche 1 bis 13, umfassend mindestens ein Bindemittel, vorzugsweise ausgewählt aus:

- Polyolen, wie Glycerin, 1,3-Propandiol, Propylenglykol, Butylenglykol, Hexylenglykol, Polyethylenglykolen,

wie PEG-8 oder Dipropylenglykol,
- Zuckern, wie Mannit, Maltodextrin, Sorbit, Xylit, Saccharose oder Glucose, und Mischungen davon.

**15.** Zusammensetzung nach einem der Ansprüche 1 bis 14, umfassend mindestens ein aus Polyolen ausgewähltes Bindemittel, vorzugsweise Propylenglykol.

**16.** Zusammensetzung nach Anspruch 14 oder 15, wobei das/die Bindemittel 1 Gew.-% bis 30 Gew.-%, bezogen auf das Gesamtgewicht der Zusammensetzung, vorzugsweise 2 Gew.-% bis 20 Gew.-%, bezogen auf das Gesamtgewicht der Zusammensetzung, und am meisten bevorzugt 5 Gew.-% bis 18 Gew.-% ausmacht.

**17.** Zusammensetzung nach einem der Ansprüche 1 bis 16, umfassend mindestens ein Cyclodextrin.

**18.** Zusammensetzung nach Anspruch 17, wobei es sich bei dem/den Cyclodextrin(en) um Oligosaccharide der Formel:

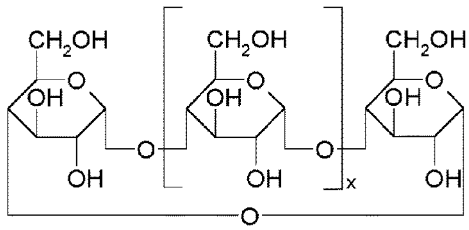

handelt, in denen x für eine Zahl gleich 4 (was einem α-Cyclodextrin entspricht) bis 5 (β-Cyclodextrin) oder bis 6 (γ-Cyclodextrin) stehen kann.

**19.** Zusammensetzung nach Anspruch 17 oder 18, wobei das/die Cyclodextrin(e) aus β-Cyclodextrin und γ-Cyclodextrin, vorzugsweise β-Cyclodextrin, ausgewählt ist/sind.

**20.** Zusammensetzung nach einem der Ansprüche 17 bis 19, wobei das/die Cyclodextrin(e) 1 Gew.-% bis 70 Gew.-%, bezogen auf das Gesamtgewicht der Zusammensetzung, vorzugsweise 2 Gew.-% bis 30 Gew.-%, bezogen auf das Gesamtgewicht der Zusammensetzung, und am meisten bevorzugt 2 Gew.-% bis 20 Gew.-% ausmacht.

**21.** Verfahren zum Reinigen oder Entfernen von Make-up von Keratinmaterialien, bei dem man eine wie in einem der Ansprüche 1 bis 20 definierte Zusammensetzung auf die Keratinmaterialien aufbringt.

**22.** Verfahren zur Herstellung einer wie in einem der Ansprüche 1 bis 20 definierten Zusammensetzung durch Nassgranulation, welches die folgenden Schritte umfasst, von denen einige gleichzeitig durchgeführt werden können:

- homogenes Mischen der pulverförmigen Rohmaterialien im Granulator;
- Herstellung der Benetzungslösung, wobei diese Lösung mindestens ein Bindemittel enthalten kann, und Überführung in den Granulator;
- Benetzen der Pulver und Mischen;
- Granulation: Ziel dieses Schrittes ist es, Pulver und Flüssigkeit durch mechanische Scherung oder durch Pulverisierung so zu vermischen, dass eine körnige Struktur entsteht;
- Trocknen zum Reduzierend des Feuchtigkeitsgehalts des Granulats;
- Kalibrierung, durch die es möglich ist, eine gute Teilchengrößenverteilung zu erhalten, indem man das Material durch ein kalibriertes Gitter oder Sieb führt;
- gegebenenfalls Zugabe von Schmiermitteln und/oder Sprengmitteln.

**Revendications**

**1.** Composition sous forme de solide anhydre, sous la forme de particules, la teneur en eau de la composition étant inférieure ou égale à 5 % en poids, comprenant au moins un tensioactif anionique choisi parmi des acides acyliséthioniques et des acyliséthionates, au moins un tensioactif anionique choisi parmi les acides acylglutamiques et les acylglutamates, au moins un tensioactif amphotère, et au moins 20 % en poids de charges par rapport au poids total de la composition.

2. Composition selon la revendication 1, dans laquelle l'au moins un tensioactif anionique est choisi parmi des acides acyliséthioniques, des acyliséthionates et leurs mélanges, la chaîne à base d'hydrocarbure du groupement acyle étant linéaire ou ramifiée, et saturée ou insaturée, et comprenant de 8 à 30 atomes de carbone, de préférence de 10 à 22 atomes de carbone et mieux encore de 12 à 18 atomes de carbone.

3. Composition selon l'une ou l'autre des revendications 1 et 2, dans laquelle les acyliséthionates sont choisis parmi les composés de formule :

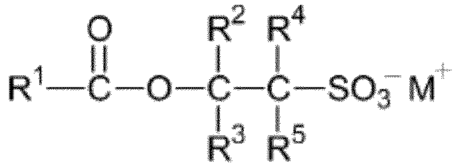

dans laquelle :

R1 représente une chaîne à base d'hydrocarbure linéaire ou ramifiée, saturée ou insaturée, comprenant de 8 à 30 atomes de carbone, de préférence de 10 à 22 atomes de carbone et mieux encore de 12 à 18 atomes de carbone, $M^+$ représente $H^+$ ou un cation, de préférence choisi parmi les ions des métaux alcalins tels que Na, Li, ou K, parmi les ions des métaux alcalino-terreux et parmi les groupes ammonium et leurs mélanges, et R2, R3, R4 et R5 représentent indépendamment un atome d'hydrogène ou un groupe alkyle, linéaire ou ramifié, de préférence linéaire, comprenant de 1 à 4 atomes de carbone.

4. Composition selon l'une des revendications 1 à 3, dans laquelle les acyliséthionates sont choisis parmi l'iséthionate de lauroyle de méthyle de sodium, l'iséthionate de méthyle de sodium, l'iséthionate de cocoyle de sodium, et leurs mélanges.

5. Composition selon l'une quelconque des revendications 1 à 4, dans laquelle les acides acyliséthioniques et/ou les acyliséthionates sont présents en une teneur en matière active allant de 1 % à 60 % en poids, de préférence de 2 % à 40 % en poids et mieux de 5 % à 30 % en poids par rapport au poids total de la composition.

6. Composition selon l'une quelconque des revendications 1 à 5, dans laquelle les acides acylglutamiques et les acylglutamates sont choisis parmi des acides acylglutamiques dans lesquels le groupement acyle comprend de 10 à 30 atomes de carbone, de préférence de 12 à 22 atomes de carbone, par exemple l'acide lauroylglutamique, l'acide myristoylglutamique, l'acide palmitoylglutamique, l'acide stéaroylglutamique, l'acide béhénoylglutamique, l'acide olivoylglutamique, l'acide cocoylglutamique et les sels de ces acides, notamment les sels de métaux alcalins tels que Na, Li ou K, de préférence Na ou K, les sels de métaux alcalino-terreux tels que Mg, ou les sels d'ammonium desdits acides.

7. Composition selon l'une quelconque des revendications 1 à 6, dans laquelle les acides acylglutamiques et les acylglutamates sont présents en une teneur en matière active allant de 1 % à 60 % en poids, de préférence de 2 % à 40 % en poids et mieux encore de 5 % à 30 % en poids par rapport au poids total de la composition.

8. Composition selon l'une des revendications 1 à 7, dans laquelle le ou les tensioactifs amphotères sont choisis parmi les N-alkyl($C_8$-$C_{22}$)bétaïnes, les N-alkyl($C_8$-$C_{22}$)sulfobétaïnes, les N-alkyl($C_8$-$C_{22}$)amidoalkyl ($C_1$-$C_6$)bétaïnes, les N-alkyl($C_8$-$C_{22}$)hydroxysultaines, les N-alkyl($C_8$-$C_{22}$)amidoalkyl ($C_1$-$C_6$) hydroxysultaines, les N-alkyl($C_8$-$C_{22}$)amphoacétates, les N-alkyl($C_8$-$C_{22}$)amphodiacétates, les N-alkyl($C_8$-$C_{22}$)amphopropionates, les N-alkyl($C_8$-$C_{22}$)amphodipropionates, les N-alkyl($C_8$-$C_{22}$)amphohydroxypropylsulfonates, le diéthylaminopropylcocoaspartamide, et leurs mélanges, de préférence parmi les N-alkyl($C_8$-$C_{20}$)bétaïnes, les N-alkyl($C_8$-$C_{20}$)amidoalkyl($C_1$-$C_6$)bétaïnes, les N-alkyl($C_8$-$C_{20}$)amphoacétates et les N-alkyl($C_8$-$C_{20}$)amphodiacétates, et leurs mélanges.

9. Composition selon l'une quelconque des revendications 1 à 8, comprenant au moins une N-alkyl($C_8$-$C_{22}$)amidoalkyl($C_1$-$C_6$)bétaïne.

10. Composition selon l'une des revendications 1 à 9, dans laquelle le ou les tensioactifs amphotères sont présents en une quantité de matière active comprise entre 0,5 % et 40 % en poids, par rapport au poids total de la composition, en particulier allant de 1 % à 30 % en poids, mieux encore de 1 % à 20 % en poids.

**11.** Composition selon l'une quelconque des revendications 1 à 10, dans laquelle les charges sont présentes en une teneur supérieure ou égale à 25 % en poids, mieux encore supérieure ou égale à 45 % en poids, par rapport au poids total de la composition.

**12.** Composition selon l'une quelconque des revendications 1 à 11, dans laquelle la quantité de charges va de 20 % à 80 % en poids, mieux encore de 30 % à 75 % en poids par rapport au poids total de la composition.

**13.** Composition selon l'une quelconque des revendications 1 à 12, comprenant au moins une charge choisie parmi les talcs, les amidons, en particulier l'amidon de riz et l'amidon de maïs, et leurs mélanges.

**14.** Composition selon l'une quelconque des revendications 1 à 13, comprenant au moins un agent liant, de préférence choisi parmi :

- les polyols tels que le glycérol, le 1,3 propanediol, le propylène glycol, le butylène glycol, l'hexylène glycol, les polyéthylène glycols tels que le PEG-8 ou le dipropylène glycol,
- les sucres, tels que le mannitol, la maltodextrine, le sorbitol, le xylitol, le saccharose ou le glucose, et leurs mélanges.

**15.** Composition selon l'une quelconque des revendications 1 à 14, comprenant au moins un agent liant choisi parmi des polyols, de préférence le propylène glycol.

**16.** Composition selon l'une ou l'autre des revendications 14 et 15, dans laquelle l'agent ou les agents liants représentent de 1 % à 30 % en poids par rapport au poids total de la composition, de préférence de 2 % à 20 % en poids par rapport au poids total de la composition, et au mieux 5 % à 18 %.

**17.** Composition selon l'une quelconque des revendications 1 à 16, comprenant au moins une cyclodextrine.

**18.** Composition selon la revendication 17, dans laquelle la ou les cyclodextrines sont des oligosaccharides de formule :

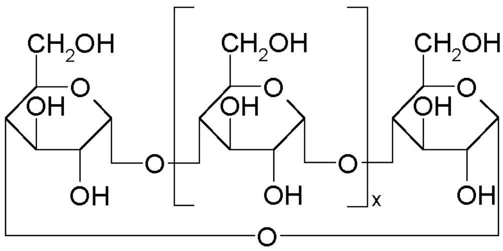

dans laquelle x peut être un nombre égal à 4 (ce qui correspond à 1'$\alpha$-cyclodextrine), à 5 ($\beta$-cyclodextrine) ou à 6 ($\gamma$-cyclodextrine).

**19.** Composition selon l'une ou l'autre des revendications 17 et 18 dans laquelle la ou les cyclodextrines sont choisies parmi la $\beta$-cyclodextrine et la $\gamma$-cyclodextrine ; de préférence la $\beta$-cyclodextrine.

**20.** Composition selon l'une quelconque des revendications 17 à 19, dans laquelle la ou les cyclodextrines représentent de 1 % à 70 % en poids par rapport au poids total de la composition, de préférence de 2 % à 30 % en poids par rapport au poids total de la composition, et au mieux 2 % à 20 %.

**21.** Procédé pour le nettoyage ou le démaquillage de matières kératiniques, une composition telle que définie à l'une quelconque des revendications 1 à 20 étant appliquée sur les matières kératiniques.

**22.** Procédé pour la préparation d'une composition telle que définie dans l'une quelconque des revendications 1 à 20 par granulation humide comprenant les étapes suivantes, certaines pouvant avoir lieu simultanément :

- mélange des matières premières pulvérulentes de manière homogène dans le granulateur ;
- préparation de la solution de mouillage, cette solution pouvant contenir au moins un agent liant, et transfert dans le granulateur ;
- mouillage des poudres et mélange ;

- granulation : l'objectif de cette étape étant de mélanger poudre et liquide pour atteindre la structure granulaire, par cisaillement mécanique ou par pulvérisation ;
- séchage, afin de diminuer la teneur en humidité des granulés ;
- calibrage, qui permet d'obtenir une bonne répartition granulométrique par passage à travers une grille calibrée ou un tamis ;
- éventuellement, ajout d'agents lubrifiants et/ou désintégrants.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2779648
- US 4330438 A
- EP 0796318 A
- WO 2009153311 A
- FR 2969925
- FR 3030269

### Non-patent literature cited in the description

- *Cosmetics and Toiletries Magazine,* 2005, vol. 120, 9-10
- The HLB system. A time-saving guide to Emulsifier Selection. ICI Americas Inc, 1984
- *J. Soc. Cosm. Chem.,* 1954, vol. 5, 249-256
- Agents de surface et émulsions [Surface agents and emulsions. **F. PUISIEUX ; M. SEILLER.** Galenica 5: Les systèmes dispersés [Galenics 5: Dispersed systems. vol. I, 153-194
- surfactant science series. Surfactants in Cosmetics. vol. 68, 134
- Kirk-Othmer's Encyclopedia of Chemical Technology. Wiley, 1979, vol. 22, 333-432
- **GRIFFIN.** *J. Soc. Cosm. Chem.,* 1954, vol. 5, 249-256